# EUROPEAN PATENT SPECIFICATION

(11) **EP 4 281 016 B1**
(45) Date of publication and mention of the grant of the patent: **23.07.2025**
(21) Application number: 22742988.3
(22) Date of filing: 11.01.2022
(51) Int. Cl.: A61F 2/66, A61F 2/00, A61F 2/42, A61F 2/50, A61F 2/60

(54) **SYSTEMS, DEVICES AND METHODS FOR MULTI-AXIAL ASSEMBLIES**
SYSTEME, VORRICHTUNGEN UND VERFAHREN FÜR MEHRACHSIGE ANORDNUNGEN
SYSTÈMES, DISPOSITIFS ET PROCÉDÉS D'ASSEMBLAGES MULTIAXIAUX

(30) Priority: 19.01.2021 US 202163139249 P; 17.06.2021 US 202117350621
(43) Date of publication of application: 29.11.2023
(73) Proprietor: Proteor USA, LLC, Tempe, AZ 85282 (US)
(72) Inventor: LIDDIARD, Steven, D., Mayfield, UT 84643 (US); BARLOW, Shelly, Ephraim, UT 84627 (US); TANGREEN, Dennis, K., Gunnison, UT 84634 (US); HEATH, Steven, J., Mayfield, UT 84643 (US); GLENN, Gregory, J., Irvine, CA 92618 (US)
(74) Representative: Reddie & Grose LLP
(86) International application number: PCT/US2022/011936
(87) International publication number: WO 2022/159304

(56) References cited:
- GB-A- 2 305 363
- US-A- 2 439 195
- US-A- 3 956 775
- US-A- 5 800 563
- US-A1- 2005 261 783
- US-A1- 2006 004 467
- US-A1- 2007 106 396
- US-A1- 2018 042 737
- US-B2- 10 857 007

## Description

### BACKGROUND

This disclosure relates generally to prosthetics for lower limb amputees, and more specifically to methods and apparatus for multi-axial assemblies to provide rotation and vertical movement to lower limb prostheses.

United States Patent Publication US 3956775 A relates to a rotator for a prosthetic ankle joint comprising a resilient body comprising an outer perimeter, a first surface, a second surface opposite the first surface, and an interior opening there between, a shaft, the shaft comprising a bore interface with at least one contact surface, a first prosthesis body coupled to the longitudinal shaft and a second prosthesis body comprising a longitudinal lumen and a flange located on an upper surface of the second prosthesis body.

US Patent Publication US 5800563 A relates to an impact reducing prosthetic pylon. UK Patent Application Publication GB 2305363 A relates to a telescopic shin prosthesis. US Patent Publication US 10857007 B2 relates to a coupling for a prosthetic device.

### BRIEF SUMMARY

Multi-axial prosthesis assemblies that include a resilient closed undulating member are used to provide vertical and rotational movement for lower limb prostheses. A shaft is located through a resilient bumper, with a first prosthetic member fixedly attached to the shaft and engages a first surface of the undulating member, and a second prosthetic member comprises a lumen to movably receive the shaft. The prosthetic members are engaged to the resilient bumper with projections that are located in the recesses of the resilient bumper and the prosthetic members and the bumper are configured so that the projections from each member are offset from the projections of the other member, which results in an undulating appearance to the outer surface of the resilient bumper.

In one embodiment, a prosthetic assembly is provided, comprising a resilient undulating body comprising an outer perimeter, a first surface, a second surface opposite the first surface, and an interior opening therebetween, a longitudinal shaft located in the interior opening of the undulating member, a first prosthesis body coupled to the longitudinal shaft and contacting the first surface of the undulating member, and a second prosthesis body comprising a longitudinal lumen and contacting the second surface of the undulating member, wherein the longitudinal shaft is movably located in the longitudinal lumen. The undulating body may comprise a first plurality of recesses on the first surface of the undulating body. The first prosthesis body may comprise a first plurality of projections configured to form a mechanical interfit with the first plurality of recesses of the undulating body. The undulating body may comprise a second plurality of recesses on the second surface of the undulating body. The second prosthesis body may comprise a second plurality of projections configured to form a mechanical interfit with the second plurality of recesses of the undulating body. The first plurality of recesses may be rotationally offset from the second plurality of recesses when no net rotational forces are acting on the undulating body. The first plurality of recesses may comprise an equal angular spacing relative to a central axis of the undulating body, and the second plurality of recesses may comprise an equal angular spacing relative to the central axis of the undulating body. The undulating body may further comprise an internal seal extending from the second surface of the undulating body that is radially offset from the outer perimeter of the undulating member and surrounding the interior opening of the undulating body. The angular spacing of the first plurality of recesses and the angular spacing of the second plurality of recesses may be 90 degrees. The first and second pluralities of recesses may be offset by 40 to 65 degrees. The first and/or second plurality of recesses may each comprise four recesses. Each recess of the first plurality of recesses and the second plurality of recesses may comprise an outer perimeter opening region, a radially inward wall opposite the outer perimeter opening, and opposing first and second side walls flanking the radially inward wall. The radially inward wall and the opposing first and second walls may comprise a U-shape on a transverse cross section through the undulating member. The recesses of the first plurality of recesses may further comprise a first surface opening region on the first surface of the undulating body, wherein the first surface opening region is contiguous with the outer perimeter opening region of the same recess, and a middle wall opposite the first surface opening region, wherein the middle wall is flanked by the first and second walls of the same recess. Each of the recesses of the second plurality of recesses may further comprise a second surface opening region on the second surface of the undulating body, wherein the second surface opening region is contiguous with the outer perimeter opening region of the same recess, and a middle wall opposite the second surface opening region, wherein the middle wall is flanked by the first and second walls of the same recess. Each recess of the first and second pluralities of recesses comprises a non-planar surface opening. The first prosthesis body may be integrally formed with the longitudinal shaft. The second prosthesis body may be configured to permit axial and rotational movement of the longitudinal shaft in the longitudinal lumen of the second prosthesis body. The prosthetic assembly may further comprise a shaft retainer removably attached to the shaft, and may be configured to resist separation of the longitudinal shaft and the second prosthesis body. The shaft retainer may comprise a removable fastener configured to removably attach to the longitudinal shaft, an annular seal configured to slidably seal the shaft retainer to the second prosthesis body, and a retaining washer with a circumferential recess in which the annular seal partially resides. The shaft retainer may further comprise a spring. The spring may be configured to maintain compression of the resilient body when the prosthetic assembly is in an unloaded state. The prosthetic assembly may further comprise an attachment pyramid. The attachment pyramid may be integrally formed with the longitudinal shaft. The second prosthesis body may further comprise a mounting interface configured to attach to a foot prosthesis. The mounting interface may comprise a plurality of lumens, each lumen configured to removably receive a fastener. The plurality of lumens may be a plurality of transverse lumens. The second prosthesis body may further comprise an annular cavity to at least partially receive the undulating body. The diameter of the interior opening of the undulating body may be greater than a diameter of the longitudinal shaft located in the interior opening of the undulating body. The longitudinal shaft may comprise a transverse stop surface located between a first end and a second end of the longitudinal shaft, and configured to displaceably abut against a corresponding stop surface of the second prosthesis body. The prosthetic assembly may further comprise a compression collar located between the first and second prosthesis bodies and configured to limit displacement of the longitudinal shaft relative to the longitudinal lumen of the second prosthesis body.

### BRIEF DESCRIPTION OF THE DRAWINGS

These and other features, aspects and advantages of the present invention will become better understood with reference to the following description, appending claims, and accompanying drawings where:
FIG. 1A is a rear elevational view of a shock rotator assembly in accordance with exemplary embodiments of the present technology;
FIG. 1B is a side elevational view of the assembly in accordance with exemplary embodiments of the present technology;
FIG. 1C is a front elevational view of the assembly in accordance with exemplary embodiments of the present technology;
FIG. 1D is a top and view of the assembly in accordance with exemplary embodiments of the present technology;
FIG. 1E is a bottom view of the assembly in accordance with exemplary embodiments of the present technology;
FIG. 1F is a front perspective view of the assembly in accordance with exemplary embodiments of the present technology;
FIG. 1G is a rear perspective view of the assembly in accordance with exemplary embodiments of the present technology;
FIG. 1H is a side cross-sectional view of the assembly in FIG. 1B in accordance with exemplary embodiments of the present technology;
FIG. 2A is a top view of the resilient body of the assembly in FIGS. 1A to 1G in accordance with exemplary embodiments of the present technology;
FIG. 2B is a side view of the resilient body of the assembly in FIGS. 1A to 1G in accordance with exemplary embodiments of the present technology;
FIG. 2C is a bottom view of the resilient body of the assembly in FIGS. 1A to 1G in accordance with exemplary embodiments of the present technology;
FIG. 2D is a top perspective view of the resilient body in accordance with exemplary embodiments of the present technology;
FIG. 2E is a cross-sectional view of the resilient body in accordance with exemplary embodiments of the present technology;
FIG. 3A is a front perspective view the exemplary assembly in FIGS. 1A to 1G attached to an exemplary foot prosthesis in accordance with exemplary embodiments of the present technology;
FIG. 3B is a side elevational view the exemplary assembly in FIGS. 1A to 1G attached to an exemplary foot prosthesis in accordance with exemplary embodiments of the present technology;
FIG. 3C is a top view of the assembly and prosthesis combination in FIG. 3A in accordance with exemplary embodiments of the present technology;
FIG. 3D is a rear view of the assembly and prosthesis combination in FIG. 3A in accordance with exemplary embodiments of the present technology;
FIG. 3E is a front view of the assembly and prosthesis combination in FIG. 3A in accordance with exemplary embodiments of the present technology;
FIG. 4A is a rear view of the exemplary assembly in FIGS. 1A to 1G, without the resilient body in accordance with exemplary embodiments of the present technology;
FIG. 4B is a side view of the exemplary assembly in FIGS. 1A to 1G, without the resilient body in accordance with exemplary embodiments of the present technology;
FIG. 4C is a front view of the exemplary assembly in FIGS. 1A to 1G, without the resilient body in accordance with exemplary embodiments of the present technology;
FIG. 4D is a cross-sectional view of the assembly in accordance with exemplary embodiments of the present technology;
FIG. 4E is a rear perspective view of the assembly in accordance with exemplary embodiments of the present technology;
FIG. 4F is a front perspective view of the assembly in accordance with exemplary embodiments of the present technology;
FIGS. 5A is a top plan view of the first housing in FIGS. 1A to 1G in accordance with exemplary embodiments of the present technology;
FIGS. 5B is a side plan view of the first housing in FIGS. 1A to 1G in accordance with exemplary embodiments of the present technology;
FIGS. 5C is a bottom plan view, respectively, of the first housing in FIGS. 1A to 1G in accordance with exemplary embodiments of the present technology;
FIG. 5D is a cross-sectional view of the first housing FIG. 5B in accordance with exemplary embodiments of the present technology;
FIG. 5E is a top perspective view of the first housing in accordance with exemplary embodiments of the present technology;
FIG. 5F is a bottom perspective view of the first housing in accordance with exemplary embodiments of the present technology;
FIG. 6A is a side elevational view of the shaft in FIGS. 1A to 1G in accordance with exemplary embodiments of the present technology;
FIG. 6B is a cross-sectional view of the shaft in accordance with exemplary embodiments of the present technology;
FIG. 6C is a top perspective view of the shaft in accordance with exemplary embodiments of the present technology;
FIG. 6D is a top plan view of the shaft in accordance with exemplary embodiments of the present technology;
FIG. 6E is a bottom plan view of the shaft in accordance with exemplary embodiments of the present technology;
FIG. 7A is a rear elevational view of the second housing in FIGS. 1A to 1G in accordance with exemplary embodiments of the present technology;
FIG. 7B is a side elevational view of the second housing in FIGS. 1A to 1G in accordance with exemplary embodiments of the present technology;
FIG. 7C front elevational view of the second housing in FIGS. 1A to 1G in accordance with exemplary embodiments of the present technology;
FIG. 7D is a cross-sectional view of the second housing FIG. 7B in accordance with exemplary embodiments of the present technology;
FIGS. 7E is a top perspective view of the second housing in accordance with exemplary embodiments of the present technology;
FIGS. 7F is a bottom perspective view the second housing in accordance with exemplary embodiments of the present technology;
FIG. 7G is a top plan view of the second housing in accordance with exemplary embodiments of the present technology;
FIG. 7H is a bottom plan view of the second housing in accordance with exemplary embodiments of the present technology;
FIG. 8A is a top plan view of the retention washer in FIGS. 1A to 1G in accordance with exemplary embodiments of the present technology;
FIG. 8B is a side elevational view of the retention washer in FIGS. 1A to 1G in accordance with exemplary embodiments of the present technology;
FIG. 8C is a cross-sectional view of the retention washer in FIG. 7B accordance with exemplary embodiments of the present technology;
FIG. 8D is a bottom plan view of the retention washer in accordance with exemplary embodiments of the present technology;
FIG. 8E is a bottom perspective view of the retention washer in accordance with exemplary embodiments of the present technology;
FIG. 8F is a top perspective view of the retention washer in accordance with exemplary embodiments of the present technology;
FIG. 9 is a cross-sectional view of another embodiment of an assembly comprising an integrated first housing and shaft in accordance with exemplary embodiments of the present technology;
FIG. 10 is a cross-sectional view of another embodiment of an assembly comprising a separate first housing and shaft in accordance with exemplary embodiments of the present technology;
FIG. 11A is a front elevational view of another embodiment of an exemplary shock rotator assembly in accordance with exemplary embodiments of the present technology;
FIG. 11B is a side cross-sectional view of the assembly in FIG. 11A in accordance with exemplary embodiments of the present technology;
FIG. 12A is a rear view of another embodiment of the assembly, shown in FIG 11A and 11B, without the resilient body; in accordance with exemplary embodiments of the present technology
FIG. 12B is a cross-sectional view of the assembly in accordance with exemplary embodiments of the present technology;
FIG. 12C is a rear perspective view of the assembly in accordance with exemplary embodiments of the present technology;
FIG. 12D is a front perspective view of the assembly in accordance with exemplary embodiments of the present technology;
FIG. 13A is a side elevational view of another embodiment of a shaft in accordance with exemplary embodiments of the present technology;
FIG. 13B is a cross-sectional view of the shaft in accordance with exemplary embodiments of the present technology;
FIG. 13C is a top perspective view of the shaft in accordance with exemplary embodiments of the present technology;
FIG. 13D is a top plan view of the shaft in accordance with exemplary embodiments of the present technology;
FIG. 13E is a bottom plan view of the shaft in accordance with exemplary embodiments of the present technology;
FIGS. 14A is a rear perspective view of another embodiment of the second housing in accordance with exemplary embodiments of the present technology;
FIG. 14B is a top view of the second housing in accordance with exemplary embodiments of the present technology;
FIG. 15 is a perspective view of the second housing shown in FIGS. 14A-B showing the annular flange in accordance with exemplary embodiments of the present technology;

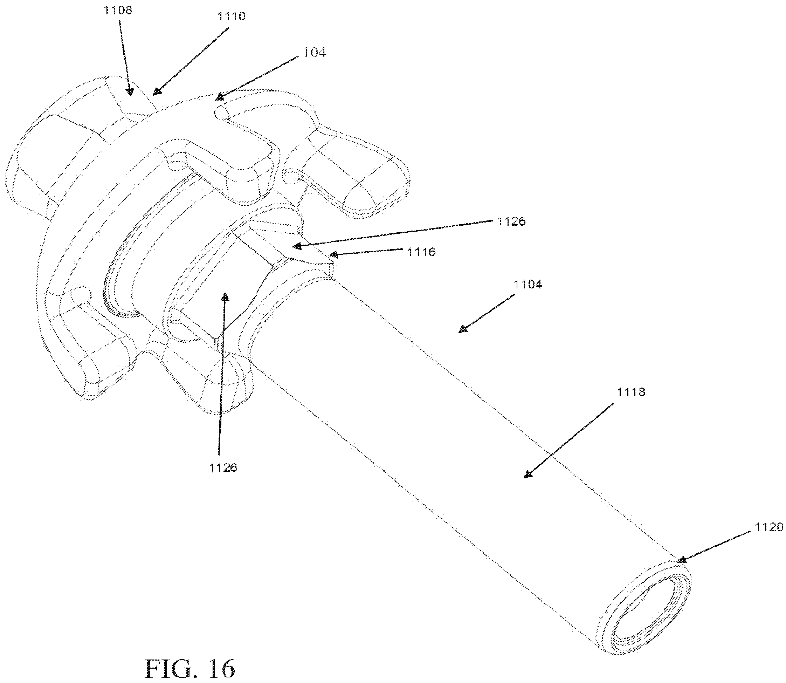

FIG. 16 is a perspective view of the first housing, shown in FIGS. 5A-D and the shaft shown in FIGS. 13A-13E in accordance with exemplary embodiments of the present technology;
FIG. 17 is a side perspective view of the second housing with a portion of the shaft placed within the lumen and the shaft in the neutral position in accordance with exemplary embodiments of the present technology;
FIG. 18 is a side perspective view of the second housing with a portion of the shaft placed within the lumen and the shaft rotated clockwise from in the neutral position in accordance with exemplary embodiments of the present technology; and
FIG. 19 is a side perspective view of the second housing with a portion of the shaft placed within the lumen and the shaft rotated counterclockwise from in the neutral position in accordance with exemplary embodiments of the present technology.

Elements and steps in the figures are illustrated for simplicity and clarity and have not necessarily been rendered according to any particular sequence. For example, steps that may be performed concurrently or in a different order are illustrated in the figures to help to improve understanding of embodiments of the present technology.

### DETAILED DESCRIPTION

The present technology may be described in terms of functional block components and various processing steps. Such functional blocks may be realized by any number of components configured to perform the specified functions and achieve the various results. For example, the present technology may be used with a prosthetic foot for various amputation types (above knee, below knee, etc.). In addition, the present technology may be practiced in conjunction with any number of materials and methods of manufacture and the system described is merely one exemplary application for the technology. In the present disclosure 1 inch is 2.54cm long, 1 pound force is 4,45 N and 1 in-lb is 0.11 Nm.

While exemplary embodiments are described herein in sufficient detail to enable those skilled in the art to practice the invention, it should be understood that other embodiments may be realized and that logical structural, material, and mechanical changes may be made without departing from the scope of the claims. Thus, the following descriptions are not intended as a limitation on the use or applicability of the invention, but instead, are provided merely to enable a full and complete description of exemplary embodiments.

The function and features of a lower limb prosthetic may be selected based on the user's ability to ambulate and to transfer from various positions from a chair or bed. For patients that are able to ambulate at a single speed on level surface, a solid ankle-cushion heel foot prosthesis, or a single-axis prosthesis may be selected, for users who are able to traverse curbs, stair and uneven surfaces, a flexible-keel foot or a multi-axial ankle/foot prosthesis may provide improved ambulation efficiency and safety. For users with greater rehabilitation potential and are able to ambulate at different speeds and traverse most environmental obstacles, a multi-axial ankle foot with vertical-loading pylon may be beneficial.

In some examples, a prosthetic assembly may be provided that permits limited axial rotation and vertical loading between two housings in which a resilient body is located. The resilient body provides limited resilient vertical loading and axial rotation as it undergoes deformation by the relative displacement and motion between the two housings. A movable shaft is attached to one of the housings, and is longitudinally and rotationally movable relative to a lumen located in the other housing in which the shaft resides. A retention member or retention assembly may be provided at the end of the shaft to releasably and movably retain the shaft in the other housing. The shaft is typically a rigid shaft that does not flex under typical loads, but in other embodiments, the shaft may comprise a resiliently flexible shaft with one or more bend regions, e.g., helical spring region that can bend away from its central longitudinal axis.

To resist substantial separation of the resilient body from the housings, the resilient body may comprise a closed shape with an interior opening in which a portion of the shaft is located. To provide increasing resistance to greater degrees of axial rotation, the housings and the resilient body may comprise complementary projections and recesses configured to resist greater amounts of rotational slippage. The complementary interface may be sized and located to also distribute rotational forces acting on the resilient body in order to reduce the concentration of forces that may increase the fracture or breakage of the resilient body. In some further embodiments, the configuration of the assembly may include projections from the first and second housings into recesses located in the resilient body. The recesses may be located around the periphery of the resilient body such that each recess is open and confluent on both a side surface and a horizontal surface of the resilient body. The angular arrangement of the recesses may be configured such that recesses are located on alternating horizontal surfaces to receive alternating projections from the two housings. This results in an undulating configuration to the side or periphery surface of the resilient body. The resilient body may further comprise one or more flanges or sealing structures to help resist water or liquid intrusion into the interior regions of assembly.

The first and second housings of the assembly may also comprise recesses or cavities to partially contain a portion of the resilient body, and an interface to fixedly or movably couple to the shaft of the assembly. In some variations, a first or upper end of the shaft is configured to fixedly attach to the first or upper housing, so that the first housing and shaft move in a fixed relationship relative to the resilient body and second housing. In other variations, the first housing and shaft may be integrally formed. Typically, the shaft is inserted through the resilient body and into a longitudinal lumen of the second or lower housing in which the shaft movably resides.

The first or upper housing, or the first or upper end of the shaft, may comprise an attachment interface to attach to a pylon or residual limb socket. The second or lower housing may comprise an attachment interface to attach the assembly to a foot prosthesis.

The second or lower end of the shaft may be accessible at the second or lower end of the second housing, and a retention member or assembly may be attached to the shaft in order to retain the shaft in the lumen of the second housing. The retention member or assembly may be detached in order to perform maintenance on the assembly or to change out the resilient body.

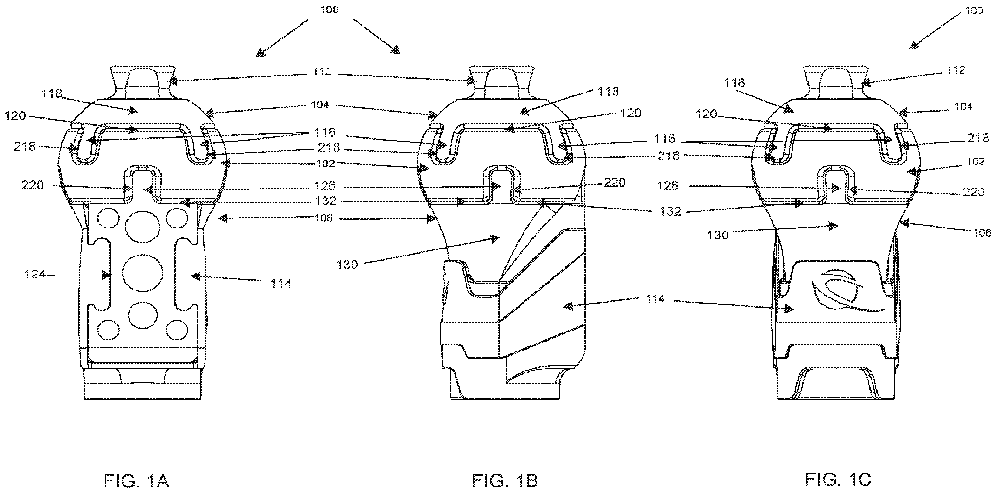

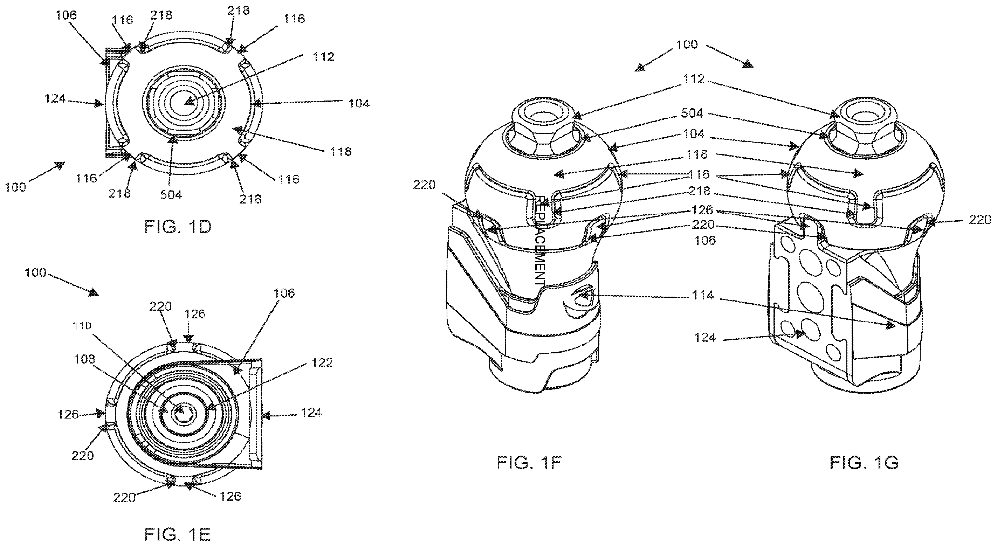

In one exemplary embodiment as described generally above, a prosthetic assembly 100 that provides vertical shock absorption and rotational movement is depicted in FIGS. 1A to 1H. The assembly 100 comprises a resilient bumper or body 102, located between a first or upper housing 104 and a second or lower housing 106. A longitudinal or vertical shaft 108 is coupled to the first housing 104, passing through the resilient body 102 and coupled to the second housing 106. A retention member or retention assembly 110 is attached to the shaft 108 to resist separation of the shaft from the second housing 106. The assembly 100 is configured to permit limited longitudinal and rotational displacement of the shaft 108 relative to the second housing 106, with the resilient body 102 providing increasing resilient resistance to increasing vertical compression and increasing rotational displacement. A pyramid attachment structure 112 is provided on the shaft 108 for attachment of the assembly 100 to a pylon or residual limb socket (not shown), while the second housing 106 is configured for attachment to a foot prosthesis. A cover piece 114 may also be provided on the assembly 100. In some variations, the cover piece 114 may provide a cosmetic/trademark function and/or a protective function to protect one or more areas of the assembly 100 from intrusion of unwanted materials (e.g., dirt, liquid) and/or inadvertent snagging of the assembly 100 with environmental objects and hazards. Although the assembly 100 described in this particular embodiment may be provided separate from a foot prosthesis, in other examples, the assembly 100 may be integrated with foot prosthesis at the point-of-manufacture.

The shaft 108 is sized to pass through a lumen 122 of the lower housing 106 such that a retention member or retention assembly 110 may be used to releasably retain the shaft 108 in the lumen 122.

The resilient body 102 of the assembly 100 may comprise a resilient material such as silicone, rubber, polyurethane, urethane, thermoplastic elastomers, thermoplastic vulcanizates (e.g., SANTOPRENE^{™} and ELASTRON^{™}), and the like. In some further embodiments, the resilient material may comprise a durometer in the range of 40A to 100A, or 50A to 90A or 60A to 90A, and may be selected based on the user's weight and/or activity level. In some examples, the resilient body 102 is selected to provide up to 1 mm, 2 mm, 3 mm, 4 mm, or 5 mm or more vertical deflection or compression, and selected to provide up to 5 degrees, 6 degrees, 7 degrees, 8 degrees, 10 degrees, 12 degrees, 14 degrees, 16 degrees, or 20 degrees of rotational deflection, or more.

In one exemplary analysis, resilient bodies of various durometers were evaluated using various loads to achieve a minimum of 2 mm of vertical deflection and a minimum of 12 degrees of angular deflection. The results of the analysis are depicted below as Table 1:

| | **Vertical Loads** | | **Torsion Loads** | | |
|---|---|---|---|---|---|
| **Resilient Body Durometer (shore A)** | **Static Test Load (lbs)** | **Vertical Deflection Actual (inches)** | **Down Force (lbs)** | **Rotation Force (in-lbs)** | **Actual Angle (°) each direction** |
| 64A | 186 | >.08 | 121 | 118 | >12 |
| 70A | 277 | >.08 | 180 | 181 | >12 |
| 77A | 360 | >.08 | 234 | 228 | >12 |
| 83A | 462 | >.08 | 300 | 220 | >12 |

In some examples, the density of the material of the resilient body may be different or lower inside the resilient body versus the exposed surfaces of the resilient body, or the exposed surfaces may comprise a different material. The resilient body may also comprise a coating, e.g. a hydrophobic or water-resistant coating to reduce water absorption into the resilient body.

As shown in FIGS. 1A to 1H, the upper housing 104 comprises a plurality of inferior projections 116 extending from its peripheral surface 118 and lower surface 120. The inferior projections 116 are located in and form a complementary interfit with the upper recesses 218 of the resilient body 102. Likewise, the lower housing 106 comprise a plurality of superior projections 126 extending from its peripheral surface 130 and upper surface 132, and are located in and form a complementary interfit with the lower recesses 220 of the resilient body 102. The lower housing 106 further comprises an attachment interface 124 which is used to attach the assembly to a foot prosthesis (not shown).

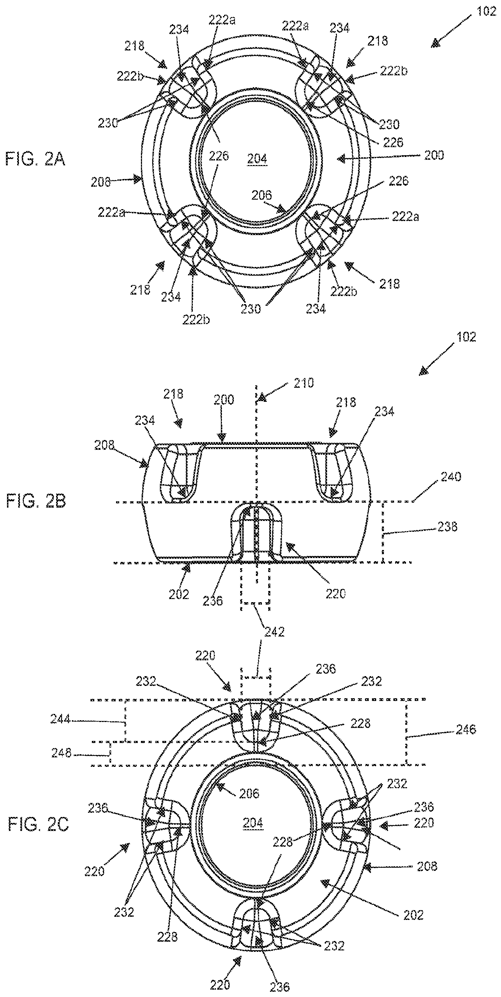

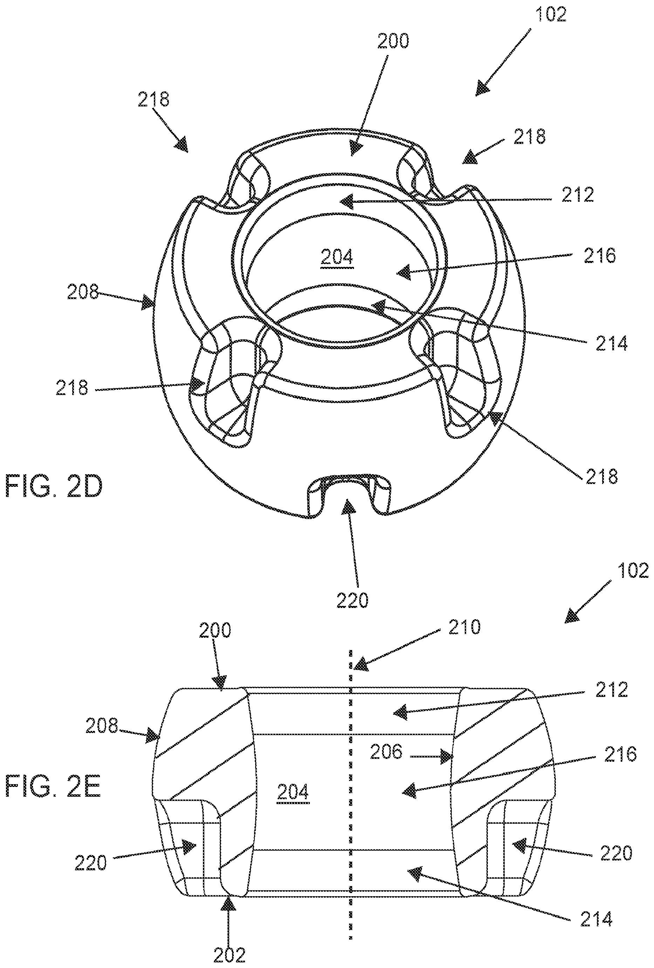

Referring to FIGS. 2A to 2E, the resilient body 102 may comprise a first or upper surface 200, a second or lower surface 202, a central lumen 204 therebetween defining an inner surface 206, and an outer lateral surface 208. Each of the upper and lower surfaces 200, 202 may comprise a generally planar configuration, but in other examples, may comprise a concave or convex configuration, or other non-planar configuration, such as a frustoconical configuration, or combination thereof. The central lumen 204 has a generally circular cross-sectional shape across its central longitudinal axis 210, but in other variations may comprise a triangular, square, rectangle, or oval shape, for example. The diameter, transverse dimension or surface area of the central lumen 204 may be constant, or may vary along the longitudinal axis 210. As depicted in exemplary resilient body 102 in FIG. 2E, the central lumen 204 may comprise a larger diameter about its upper and lower regions 212, 214, but a smaller diameter about the middle region 216. In this example, the transitions along the regions 212, 214, 216 are gradual, such that the inner surface 206 comprise a convex configuration on the cross-sectional view in FIG. 2E, but in other examples, the transitions may be abrupt, with a stepped surface configuration, for example. Similarly, the outer surface 208 of the resilient body 102 also comprises a convex shape on cross-section, but in other examples, may comprise a concave, linear, frustoconical or other shape. The larger diameter may be in the range of 0.4 inches to 3.0 inches, 0.6 inches to 2.0 inches or 0.8 inches to 1.3 inches. The smaller diameter may be in the range of 0.20 inches to 2.8 inches, 0.4 inches to 1.8 inches or 0.7 inches to 1.2 inches, and the average diameter may be in the range of 0.3 inches to 2.9 inches, 0.5 inches to 1.9 inches or 0.75 inches to 1.25 inches. The central lumen 204 may be sized such that its inner surface 206 is spaced apart and not in contact with the shaft 108 during typical usage. In some variations, some radially inward bulging of the inner surface 206 may be expected during vertical compression of the resilient body 102, and thus the dimension of the central lumen 204 may be size sufficiently to reduce the likelihood that the inner surface 206 will contact the shaft 108 during compression. The annular gap between the inner surface 206 and the shaft 108 may be in the range of 0.001 inches to 1.0 inches, 0.02 inches to 0.5 inches or 0.03 inches to 0.25 inches. The average diameter or maximum transverse dimension of the resilient body 102 across opposite sides of the outer surface 208 may be in the range of .7 inches to 3.5 inches, 1 inches to 2.5 inches or 1.5 inches to 2.25 inches.

Referring still to FIGS. 2A to 2E, the exemplary resilient body 102 comprise a set of upper recesses 218 and a set of lower recesses 220. The recesses in each set of recesses may comprise the same recess shape or configuration, and may be equally spaced apart, though between the upper recesses 218 and the lower recesses 220, the angular orientations are offset such that the angular position of each upper recess 218 is spaced equally apart from the adjacent lower recesses 220, as is each lower recess 220 is spaced equally apart from the adjacent upper recesses 218. In this example, each set of recesses 218, 220 comprises four recesses that are spaced 90 degrees apart around the resilient body, and are offset by 45 degrees between the two sets of recesses 218, 220. This permits the resilient body 102 to be assembled or serviced without requiring a particular angular alignment or top/bottom orientation, which may simplify assembly and replacement, and may reduce premature wear. In other examples, however, the resilient body 102 may not have such symmetry and therefore may be limited to a single or smaller number of positions/orientations. In other variations, for example, one or more recesses may comprise a different size, shape or spacing than the other recesses of the same set, and/or the number of recesses between the two sets of recesses may be different. In other examples, the number of recesses in each set of recess may be in the range of 2 to 5 recesses, 3 to 4 recesses, or 3 to 5 recesses.

Referring still to the recesses 218, 220 depicted in FIGS. 2A-2E, the recesses comprise openings 222, 224 that are angled or non-planar, with portions 222a, 224a of the openings 222, 224 on the upper and lower surfaces 200, 202 of the resilient body 102, respectively, that are contiguous with portions 222b, 224b of the openings 222, 224 that are located on the outer surface 208. Thus, each opening 222, 224 has a non-planar configuration with a boundary located on the outer surface 208 and either upper or lower surfaces, and where the different portions 222a, 222b, 224a, 224b are generally orthogonal to each other. In this particular embodiment, the recesses 218, 220 comprise an inner wall 226, 228 such that the recesses 218, 220 do not open to the central lumen 204 of the resilient body 102. This configuration may reduce the intrusion of debris or foreign matter into the device during use, which may interfere with smooth movement of the shaft 108 with the lumen 204 of the lower housing 106. This configuration may also shift, distribute or transfer torque exerted by the upper and lower housings 104, 106 from the inner regions to the outer regions of the resilient body 102, which will reduce torque forces acting on the resilient body 102 and may prolong its usable life being requiring replacement.

Each of the recesses 218, 220 also comprise side walls 230, 232 and end walls 234, 236. As shown in FIGS. 2A-2E, the transitions between the walls 226, 228, 230, 232, 234, 236 and with the upper and lower surfaces 200, 202 of the resilient body 102 may be rounded rather than sharply angled. This may reduce the concentration of forces transferred from the lower and upper extensions of the upper and lower housings 104, 106, or otherwise distribute the transferred forces or stresses throughout the resilient body 102, which may reduce the risk of fracture or tearing, thereby extending the life of the resilient body 102. The height 238 of each recess 218, 220 may be characterized by as the distance between either upper or lower surfaces 200, 202 of the resilient body 102 to the corresponding end wall 234, 236, as best seen in FIG. 2B. The height 238 may be in the range of 0.1 inches to 3 inches, 0.2 inches to 1.5 inches or 0.3 inches to 1 inches. The height 238 of each recess 218, 220 may also be characterized as a percentage of the height of the resilient body 102, e.g., the distance between the upper and lower surfaces 200, 202. In the particular embodiment depicted in FIG. 2B, each of the recesses 218, 220 have a relative height 238 of 50% of the resilient body 102, each with an end wall 234, 236 at the midplane 240 of the resilient body 102. In other variations, the recesses may have a relative height 238 in the range of 20% to 80%, 30% to 70%, 40% to 60%, or 50% to 70%, for example. The width 242 of each recess 218, 220 may be the average width or the maximum width based on the distance between the sidewalls, and may be in the range of 0.04 inches to 1.5 inches, 0.125 inches to 1 inches or 0.15 inches to 0.5 inches. The radial depth 244 of the recesses 218, 220 may be characterized by the distance between the outer surface 208 and the inner walls 226, 228 of the recesses 218, 220, as depicted in FIG. 2C, and may be in the range of 0.04 inches to 1.5 inches, 0.1 inches to 1 inches or 0.2 inches to 0.5 inches. In some variations, the width of each recess 218, 220 between the side walls 230, 232 may be tapered in a radially inward direction, e.g., each side wall 230, 232 is located in a plane intersecting the center longitudinal axis 210 of the resilient body 102. In other variations, the angles of the side walls 230, 232 relative to the plane may vary from about ±1 to ±5 degrees, ±2 to ±10 degrees, or ±4 to ±20 degrees, relative to the plane intersecting the center longitudinal axis 210, for example. In some further variations, the angles of the side walls 230, 232 may be altered such that the side walls 230, 232 are parallel, or where the width of each recess 218, 220 is constant or increases toward the center axis, so that during rotation, the resilient member has a radial displacement force component that drives the resilient member towards the center line. This is in contrast to side wall angles that generate a radial outward displacement force from portions of the resilient body 102 being squeezed between, which may reduce the working life of the resilient member. The radial depth 244 of the recesses 218, 220 may also be characterized as a relative percentage of the radial or annular distance 246 between the inner and outer surfaces 206, 208 of the resilient body 102, also depicted in FIG. 2C. The relative radial depth 244 may be in the range of 30% to 90%, 40% to 80% or 50% to 80%, for example. The radial thickness 248 of the inner walls 226, 228 may also be characterized as the radial distance between the inner walls 226, 228 and the inner surface 206 of the central lumen 204. The radial thickness 248 may be in the range of 0.04 inches to 2.0 inches, 0.07 inches to 1 inches or 0.1 inches to 0.5 inches, and may also be characterized as a relative thickness 248 as a percentage of the annular distance 246. The relative thickness 248 may be in the range of 10% to 70%, 20% to 60%, or 20% to 50%, for example. These dimensions may be measured based on the average dimension and exclude the curved regions of the recesses 218, 220 at the transitions between different walls and surfaces.

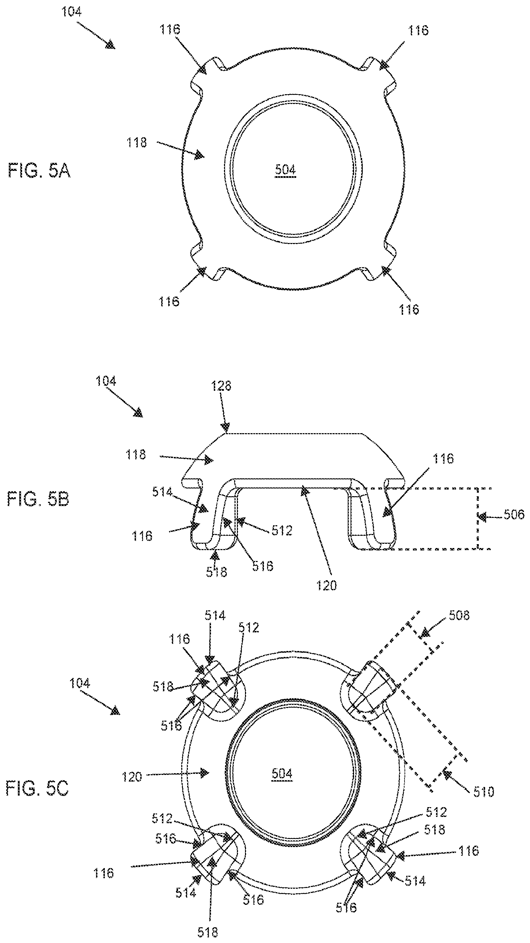

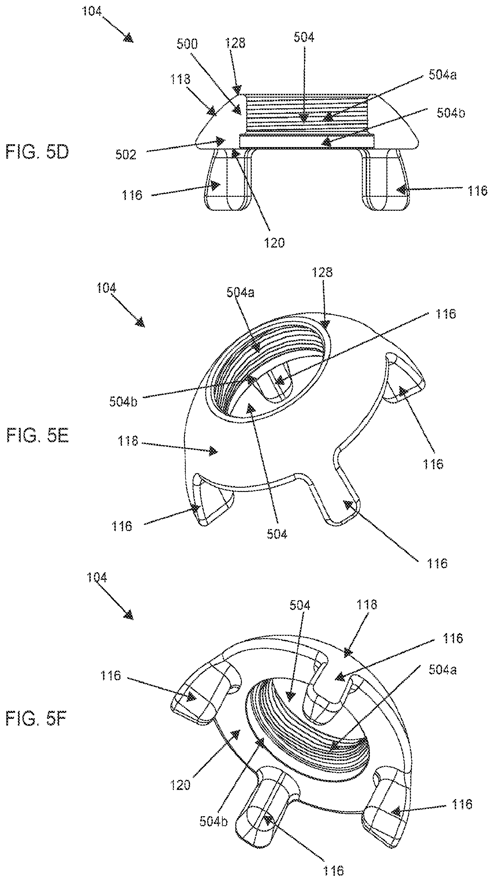

FIGS. 5A to 5F depicts additional details of the upper housing 104 of the assembly 100 depicted in FIGS. 1A to 1H. As noted previously, the upper housing 104 comprises a plurality of inferior projections 116 extending from its peripheral surface 118 and lower surface 120. When assembled, the inferior projections 116 are located in and form a complementary interfit with the upper recesses 218 of the resilient body 102. In this exemplary embodiment, the peripheral surface 118 comprises a convex, tapered shape with a larger diameter or transverse dimension in the lower region 500 closer to the inferior projections 116 and lower surface 120, and a reduced diameter or transverse dimension in the upper region 502 of the upper housing 104. Because of the taper, the upper surface 128 has a minimal or substantially reduced surface area as compared to the lower surface 120. In other variations of the upper housing 104, however, the peripheral surface 118 may not be as tapered or may comprise a generally cylindrical in shape, or comprise a non-circular or polygonal shape with linear or vertically oriented surface.

The average length 506, average width 508 and average radial depth 510 of each inferior projection 116 may be complementary to the sizes of the corresponding recesses 218. In some variations, the dimensions 506, 508, 510 of each inferior projection 116 may be slightly smaller or larger than the dimensions 238, 242, 244 of the recesses 218. In some examples, the inner surface 512 of each inferior projection 116 may have a generally vertical orientation or parallel orientation relative to the center longitudinal axis 210 of the upper housing 104. The outer surface 514 of each inferior projection 116 may comprise a taper that is in continuity with the taper and/or curvature of the peripheral surface 118, and may be flush, recessed, or protrude from the portion of the recess 218 on the outer surface 208 of the resilient body 102. Like the recesses 218, the inferior projections 116 may comprise rounded edges between the transitions of the lower surface 120, inner surface 512, outer surface 514, and side walls 516 and end wall 518.

The upper housing 104 further comprises a central lumen 504 between the lower and upper surfaces 120, 128. The central lumen 504 is configured to receive the longitudinal shaft 108 of the assembly 100. As illustrated in FIG. 5D, the central lumen 504 comprises a reduced dimension upper region 504a, and enlarged dimension lower region 504b, with a stepped surface 504c therebetween. The upper region 504a may comprise a threaded interface for attaching the shaft 108 to the upper housing 104, though in the variations the lower region 504b or both regions 504a, 504b may comprise threads, or other type of mount (e.g. bayonet mount) may be provided between the upper housing and shaft. A glue, such as an acrylate or cyanoacrylate may also be added to the threaded interface, to resist decoupling from torsional forces acting through the shaft.

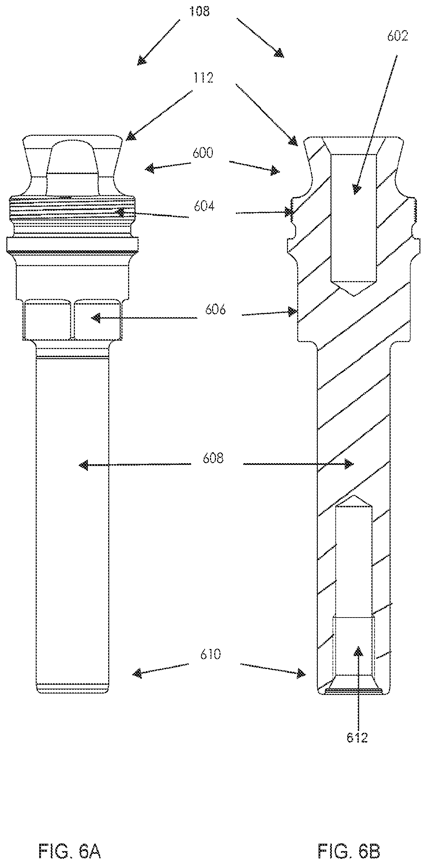

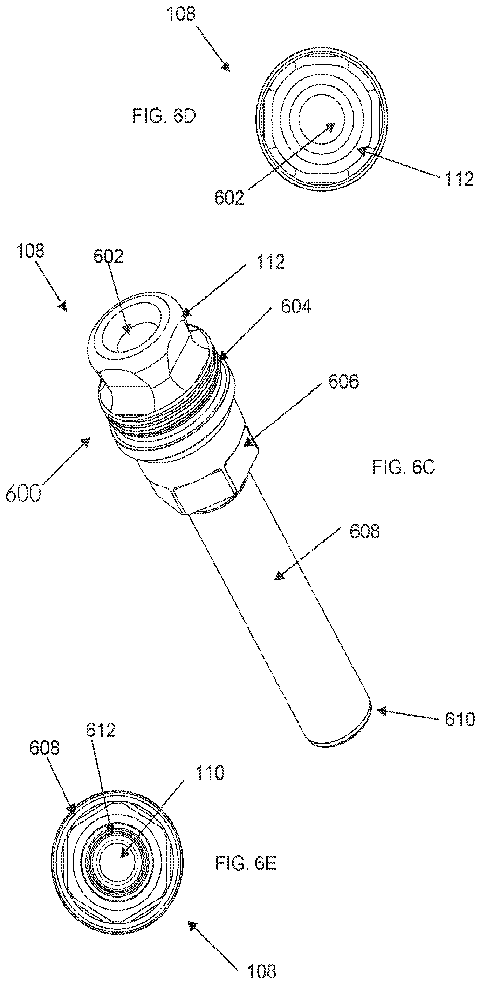

As illustrated in FIGS. 1A to 1H and 6A to 6C, the pyramid attachment structure 112 is provided on the shaft 108 for attachment of the assembly 100 to a pylon or residual limb socket. The pyramid 112 typically comprises an industry standard four-sided configuration, but in other examples, may comprise an alternative or proprietary design. The pyramid configuration may be changed by using a different shaft with a different pyramid configuration. Referring to FIGS. 6A to 6C, the pyramid 112 is located at a first end 600 of the shaft 108 and may include a threaded lumen 602 to facilitate attachment of the pyramid 112. Next to the pyramid 112 is an attachment region or interface 604 of the shaft 108 that forms a complementary interfit with the central lumen 504 of the upper housing 104. This may be a threaded interface as depicted, or a bayonet mount or other type of mechanical interfit or friction fit as noted above. As depicted in FIGS. 1A to 1H, the shaft 108 may be configured such that when assembled with the upper housing 104, the pyramid 112 protrudes from the upper surface 128 of the upper housing 104. Adjacent to the attachment interface 604 of the shaft 108 may be a tool interface 606, which may be used to grip the shaft 108 with a wrench or pliers or other tool when coupling or decoupling the shaft 108 and the upper housing 104. Although the tool interface 606 depicted in FIGS. 6A to 6C is a hexagonal interface, in other variations, the tool interface 606 may be square or rectangular or other polygonal shape, or may comprise a lumen in which a torque bar may be inserted to facilitate rotational coupling and decoupling of the shaft 108 and upper housing 104.

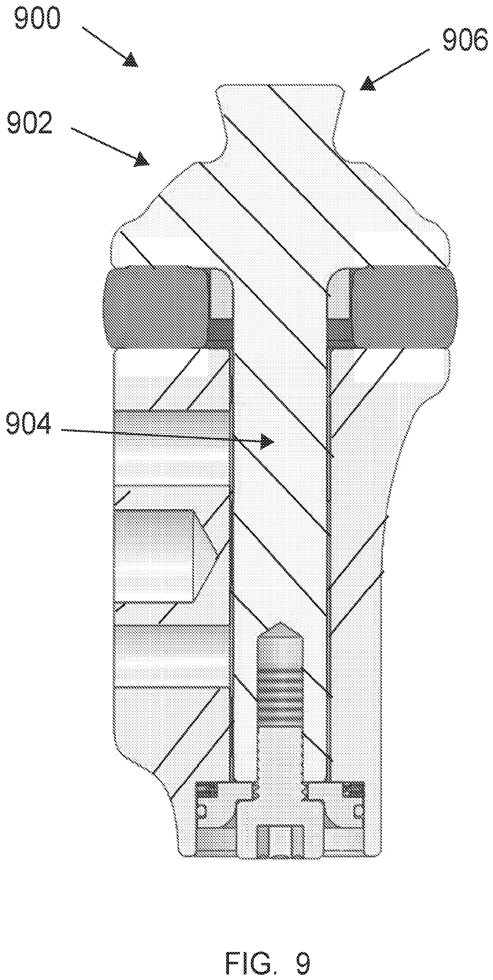

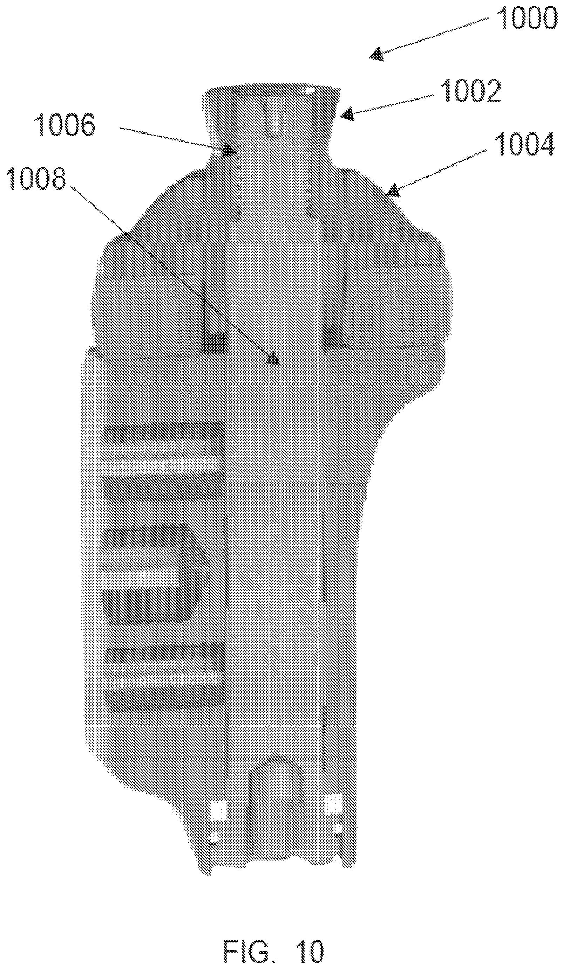

In still other variations of the assembly 900, the upper housing 902 and the shaft 904 and pyramid 906 may be integrally formed as a monolithic component, as shown in FIG. 9. In still other examples, as illustrated in FIG. 10, the assembly 1000 may comprise a pyramid structure 1002 that is integrally formed with the upper housing 1004 of the assembly 1000, but with a recess or lumen 1006 in the upper housing 1004 to couple to a shaft 1008. In this particular embodiment, the lumen 1006 of the upper housing 1004 is open at both ends and is located through the pyramid 1002 and the main body 1008 of the upper housing 1004, but in other variations, the lumen 1006 may be close-ended and with only a lower opening 1010 of the lumen, with the upper opening 1012 in the pyramid 1002.

Referring back to FIGS. 6A to 6E, adjacent or inferior to the tool interface 606 of the shaft 108 is the body 608 of the shaft 108, which is configured to reside and move in the lumen 122 of the lower housing 106 when assembled. The length of the body 608 of the shaft 108 may be in the range of 1.0 inches to 7.0 inches, 2.0 inches to 5.0 inches or 2.0 inches to 4.0 inches. The diameter or cross-sectional dimension of the shaft 108 may be in the range of 0.12 inches to 1.5 inches, 0.25 inches to 1.25 inches or 0.3 inches to 0.9 inches. Different lengths of the shaft 108 may also be provided in order to accommodate different patient preferences, height, and functional levels, with corresponding different heights of the resilient body 102.

The second or lower end 610 of the shaft 108 is sized and configured to extend out from the lumen 122 of the lower housing 106. A retention member or assembly 110 may be attached to the second end 610 to resist pullout of the shaft 108 from the lower housing 106, but may be configured to permit some vertical displacement of the shaft 108 within the lumen 122. This acts as a shock absorber as the upper housing 104 and lower housing 106 resiliently compress the resilient body 102. In this particular example, the retention assembly 110 is attachable to the second end 610 of the shaft 108 by a closed threaded lumen 612, but in other variations, may be attached via a clevis pin or other coupling interface. The retention assembly 110 is also configured to permit the shaft 108 to rotate within the lumen 122 and thereby to permit axial rotation. In the particular examples depicted in FIGS. 1A to 1H, the axial rotation is limited by the increasing resistance to rotation provided by rotational compression of the resilient body 102 between the inferior and superior projections 116, 126. In other variations, however, the retention member or assembly 110, the shaft 108 and/or the lower housing 106 may be configured with one or more complementary flanges and recesses to provide a hard limit angle limit to the rotation range.

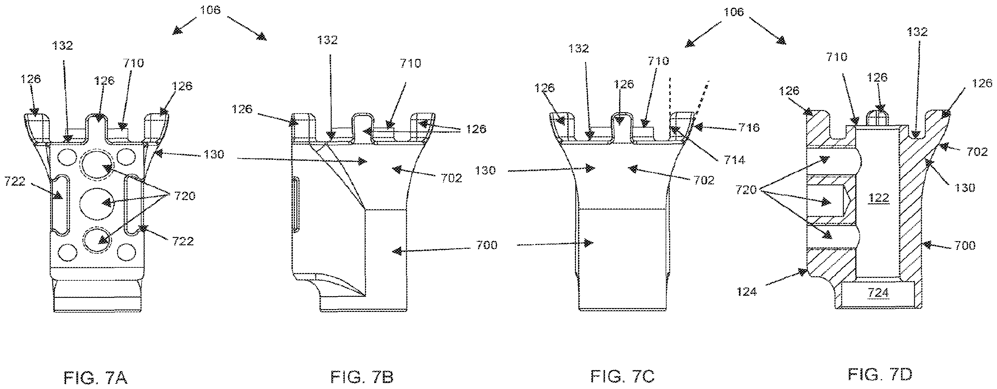

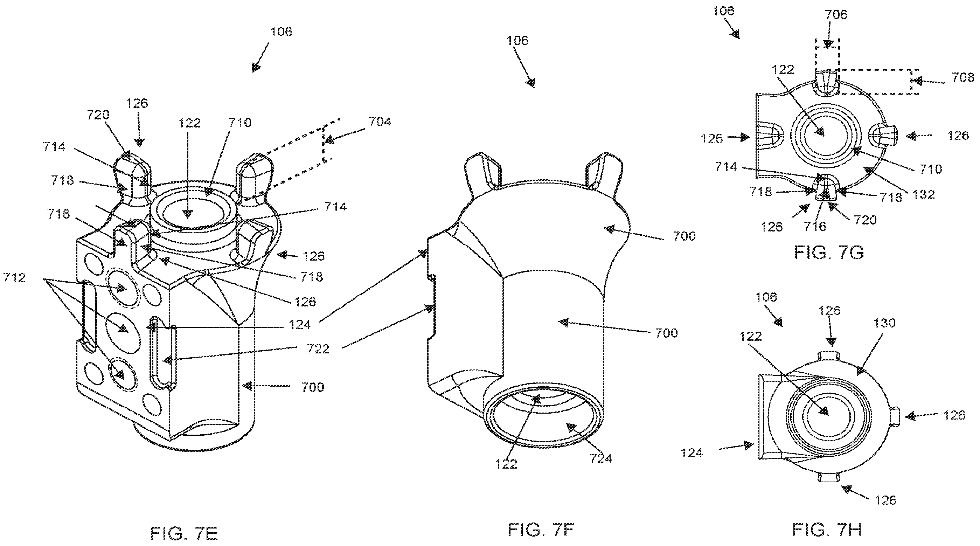

Referring now to the lower housing 106, which is detailed in FIGS. 7A to 7H, as noted previously, the lower housing 106 comprise a plurality of superior projections 126 extending from its peripheral surface 130 and upper surface 132. The superior projections 126 are positioned and configured to form a complementary interfit with the lower recesses 220 of the resilient body 102. The lower housing 106 further comprises a longitudinal lumen 122 to receive the shaft 108. The lower housing 106 comprises a main body 700 in which the lumen 122 resides, and also includes the prosthesis attachment interface 124 described earlier. The lumen 122 may include a lubricant or lubricious coating to facilitate longitudinal and rotational movement of the shaft 108 therein, but in some examples, a tubular bearing may be provided to facilitate such movement, such as a SPRINGGLIDE^{™} bearing (St. Gobain; Courbevoie, France).

Like the inferior projections 116 of the upper housing 104, the average length 704, average width 706 and average radial depth 708 of each superior projection 126 may be complementary to the sizes of the corresponding recesses 220 of the resilient body 102. In some variations, the dimensions 704, 706, 708 of each superior projection 126 may be slightly smaller or larger than the dimensions 704, 706, 708 of the recesses 220. In some examples, as depicted in FIG. 7C, the inner surface 714 of each superior projection 126 may have a generally vertical orientation or parallel orientation relative to the longitudinal axis of the upper housing 104. The outer surface 716 of each superior projection 126 may comprise a taper that is in continuity with the taper and/or curvature of the peripheral surface 132, and may be flush, recessed, or protrude from the portion of the recess 220 on the outer surface 208 of the resilient body 102. Like the recesses 220, the superior projections 126 may comprise rounded edges between the transitions of the superior surface 132 of the lower housing 106, and the inner surface 714, outer surface 716, side walls 718 and end wall 720 of the superior projections 126.

The superior surface 132 of the lower housing 106 may comprise a similar configuration as the lower surface 120 of the upper housing 104 but with an angular offset to the projections 126. In the embodiment depicted in FIGS. 7A to 7E, however, the superior surface 132 further comprises an annular projection or flange 710. The annular flange 710 is spaced radially inward from the peripheral surface 130 and the superior projections 126, surrounding the longitudinal lumen 122 of the lower housing 106. This flange 710 may be configured to insert or reside inside the central lumen 204 of the resilient body 102. In some variations, the annular flange 710 may reduce the risk of eccentric displacement of the resilient body 102 during various compression and rotational movements, and may also limit the radially inward bulging of the inner surface 206 during vertical compression, and/or may act as barrier reduce the intrusion of debris and liquid into the lumen 122 of the lower housing 106. The flange 710 also provides additional support for longer tubular bearings that might be used in the lumen 122. The use of a longer bearing may augment or reduce resistance that may be generated by off-axis forces or forces transverse to the longitudinal shaft and lumen. This may also improve bearing life and tactile prosthesis response. In embodiments comprising a tubular bearing, the ratio of the bearing length to the bearing inner diameter may in the range of 1.5:1 and 10:1, or 2:1 to 6:1 or 3:1 to 5:1. The flange 710 also allows the resilient member to be placed lower in the overall prosthesis, relative to the lumen 122, which can shorten the build height of the prosthesis, which allows the use of the prosthesis across a greater range of residual limb lengths. Depending on the height of the annular flange 710, the flange 710 may also provide a hard compression stop if the amount of vertical compression results in the annular flange 710 abutting against the inferior surface 120 of the upper housing 104. In some variations, the height of the annular flange 710 is the range of 0.02 inches to 1.5 inches, 0.1 inches to 0.5 inches or 0.12 inches to 0.3 inches. The wall thickness of the flange 710 may be in the range of 0.04 inches to 0.5 inches, 0.07 inches to 0.3 inches or 0.1 inches to 0.2 inches. The inner diameter may be 0.25 inches to 1.5 inches, 0.3 inches to 1.0 inches or 0.5 inches to 0.75 inches, and the outer diameter may be 0.3 inches to 2.0 inches, 0.4 inches to 1.5 inches or 0.5 inches to 1.0 inches.

The peripheral surface 130 of the lower housing 106 may also comprise a convex, tapered shape with a larger diameter or transverse dimension in the upper anterior region 702. The attachment interface 124 of the lower housing 106 may comprise a flat, vertically planar surface to facilitate attachment of the lower housing 106 to a foot prosthesis, but in other variations, the lower housing 106 may comprise an angled or horizontal region to facilitate attachment to foot prostheses with a corresponding angled or horizontal attachment site.

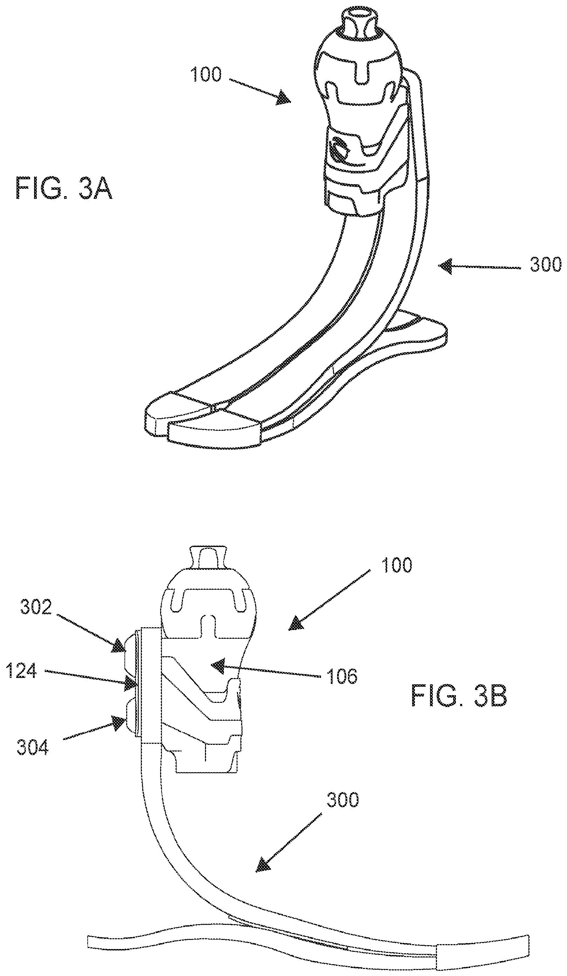

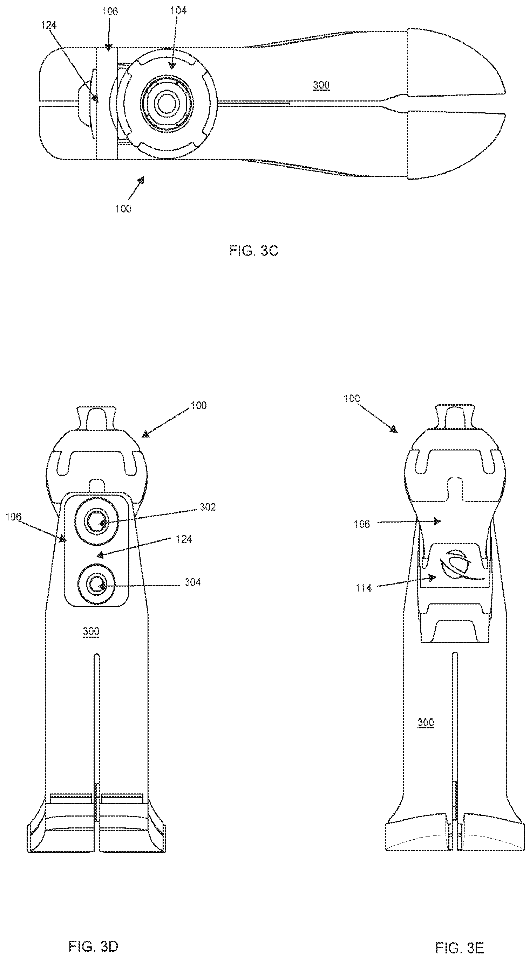

The attachment interface 124 of the lower housing 106 comprise one or more threaded lumens 712 to facilitate attachment of the lower housing 106 to a foot prosthesis using screws, bolts or other fasteners. In FIGS. 3A to 3E, the assembly 100 is attached to a foot prosthesis 300 with a vertically mounted attachment interface, using bolts 302, 304.

As depicted in FIG. 7A, the attachment interface 124 of the lower housing 106 may also comprise cover attachment sites 722 which facilitate the attachment of cosmetic covers 114 to the body 700 of the lower housing 106. The lumen 122 of the lower housing 106 may comprise a retention cavity 724 in which the retention assembly 110 resides. In other variations, however, a retention cavity is not provided such that the retention assembly 110 may protrude from the lumen 122 and the lower housing 106.

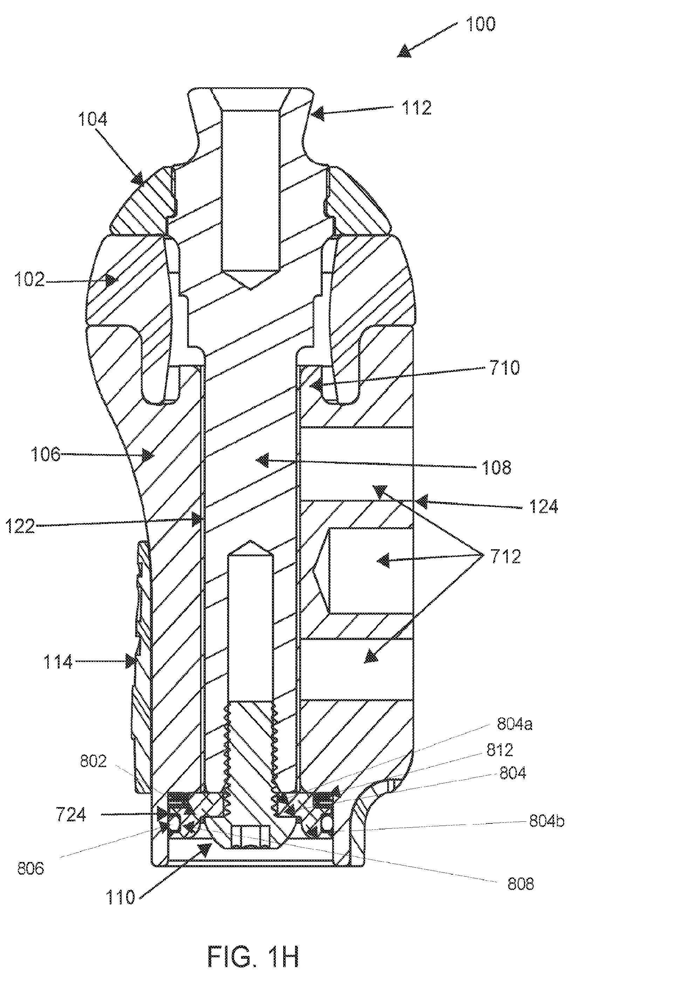

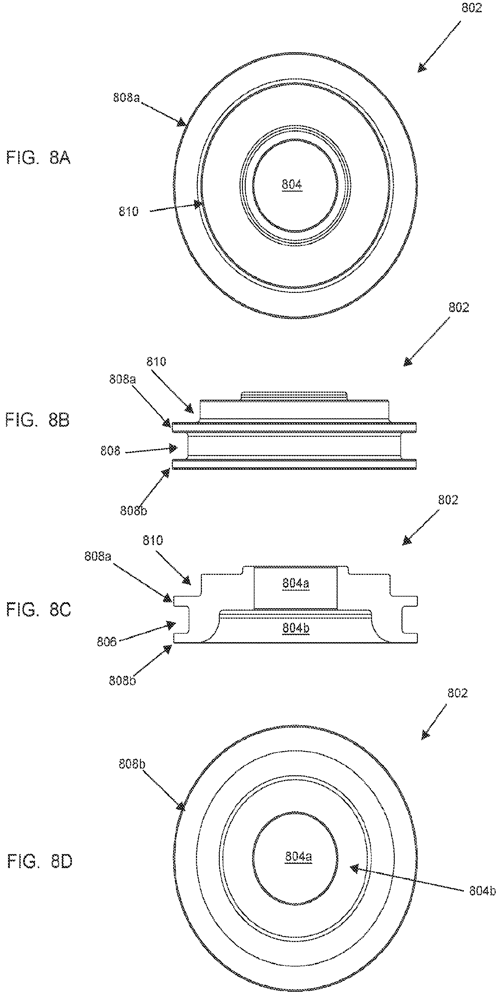

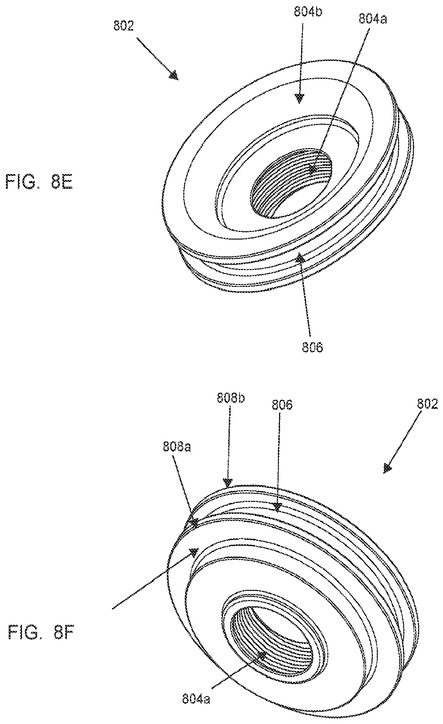

As noted previously, the retention member or assembly 110 may be attached to the shaft 108 using the threaded lumen 612 at the lower end of the shaft 108, as depicted in FIG. 1H. The retention assembly 110 may comprise a bolt 800 or other type of fastener, and a retention washer 802 which is movable in the retention cavity 724. The retention washer 802 resists further upward displacement of the shaft 108 once it abuts the superior surface of the retention cavity 724. The retention washer 802 comprises a washer cavity 804 to receive the bolt 800, and may include a reduced diameter shaft cavity 804a and an enlarged head cavity 804b which allows the bolt 800 to have a recessed position partially in the retention washer 802 when attached to the shaft 108. To reduce the risk of debris and liquid interfering with the movement of the shaft 108 in the lumen 122 of the lower housing 106, an O-ring or annular sliding seal 806 may be provided on the retention washer 802. The seal 806 is maintained in a slidable arrangement with the retention cavity 724 by a seal recess 808 on the retention washer 802, bound by recess walls 808a and 808b, as shown in FIGS. 8A to 8F. The retention washer 802 may also comprise a spring recess 810 that is superior or proximal to the recess wall 808a. Referring back to FIG. 1H, the spring recess 810 permits the positioning of a spring 812 which can be used to provide some limited inferior bias to the shaft 108 and may keep the resilient body 102 in a minimum amount of compression to the assembly 100. This minimum compression may be useful if or as the resilient body 102 undergoes any permanent compression or compression set during use. The spring 812 may be a helical spring or a wave washer, for example. The seal 804 may comprise silicone, Buna-N rubber, and Fluorinated elastomer such as VITON^{™} (Chemours; Wilmington, DE).

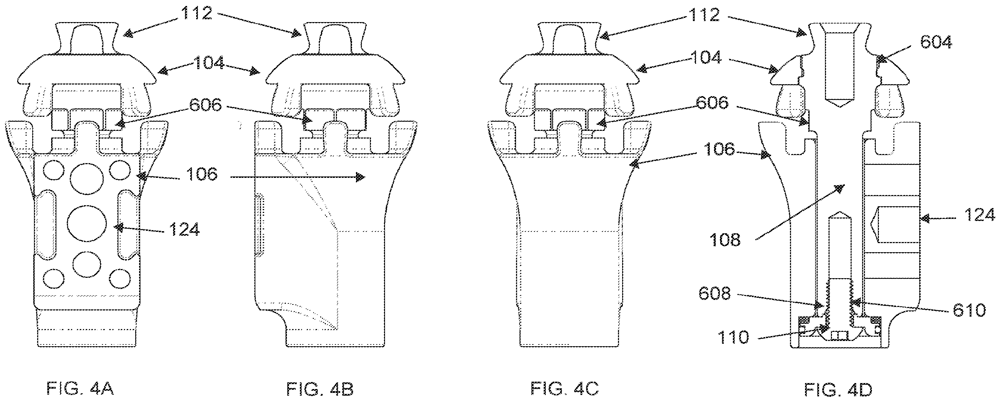

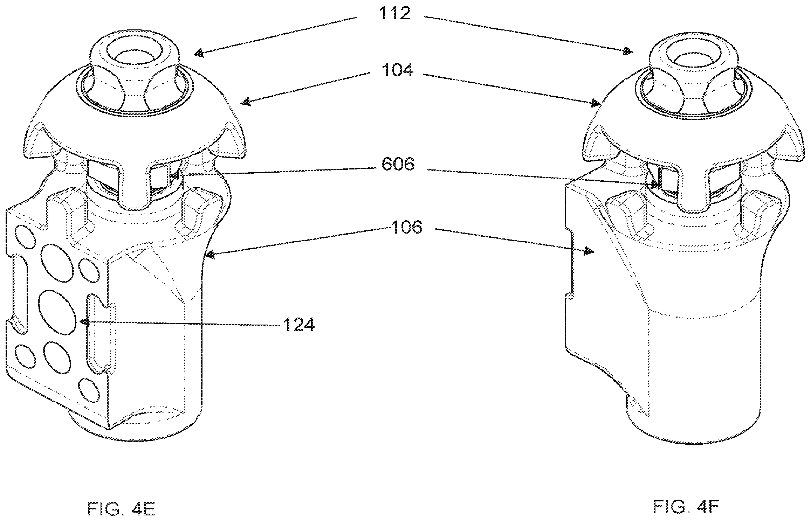

FIGS. 4A to 4F illustrate the assembled configuration of the upper housing 104, lower housing 106 and shaft 108, without the resilient body 102. The shaft 108 may be configured such that the tool interface 610 is located generally at the level of the longitudinal location of the resilient body 102. The gap or distance between the lower surface 120 of the upper housing 104 and the superior surface 132 of the lower housing 106 may be equal to the unstrained height of the resilient body 102. In other examples, the gap or distance may be smaller than the unstrained height of the resilient body 102, such that when assembled, the upper and lower housings 104, 106 place the resilient body 102 in vertical compression at baseline. This baseline compressed configuration may make the haptic feel of the assembly to be more linear or predictable compared to a baseline configuration that is not compressed or where the housing gap is greater than the unstrained height of the resilient body 102.

The upper housing 104, lower housing 106, shaft 108 and/or cover piece 114 may comprise stainless steel (e.g. 17-4 stainless steel), titanium or cobalt chromium, aluminum or other metal, and anodized variants thereof, but in other examples may comprise a rigid polymer, ceramic or a composite thereof.

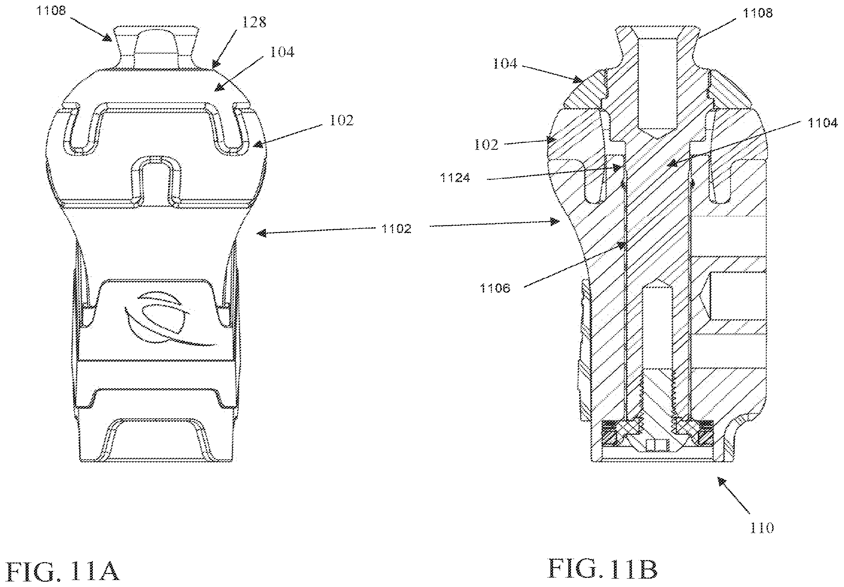

In another exemplary embodiment, shown in FIG 11A and 11B, a prosthetic assembly 1100 that provides vertical shock absorption and rotational movement is depicted in FIGS. 11A and 11B. The assembly 1100 comprises many components similar to the prosthetic assembly 100 described above and the similar components will not be discussed in detail below. The assembly 1100 comprises a resilient bumper or body 102, located between a first or upper housing 104 and a second or lower housing 1102. A longitudinal or vertical shaft 1104 is coupled to the first housing 104, passing through the resilient body 102 and coupled to the lower housing 1102. A retention member or retention assembly 110 is attached to the shaft 1104 to resist separation of the shaft 1104 from the lower housing 1102. The assembly 1100 is configured to permit limited longitudinal and rotational displacement of the shaft 1104 relative to the lower housing 1102, with the resilient body 102 providing increasing resilient resistance to increasing vertical compression and increasing rotational displacement.

The shaft 1104 is sized to pass through a lumen 1106 of the lower housing 1102 such that a retention member or retention assembly 110 may be used to releasably retain the shaft 1104 in the lumen 1106.

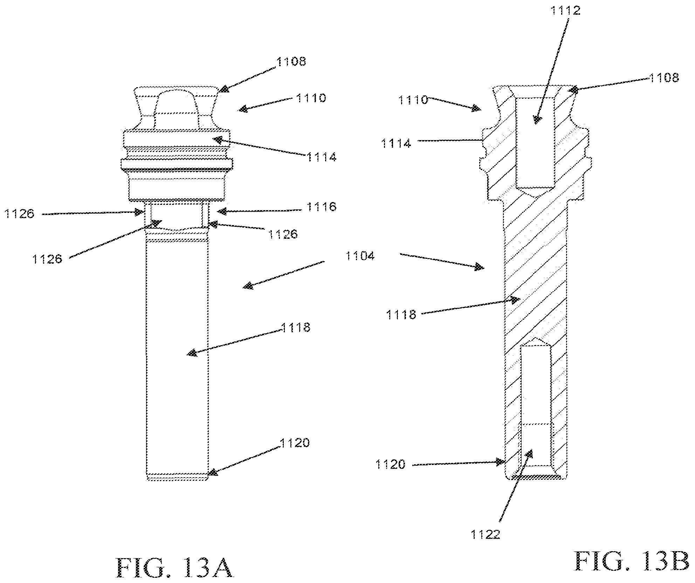

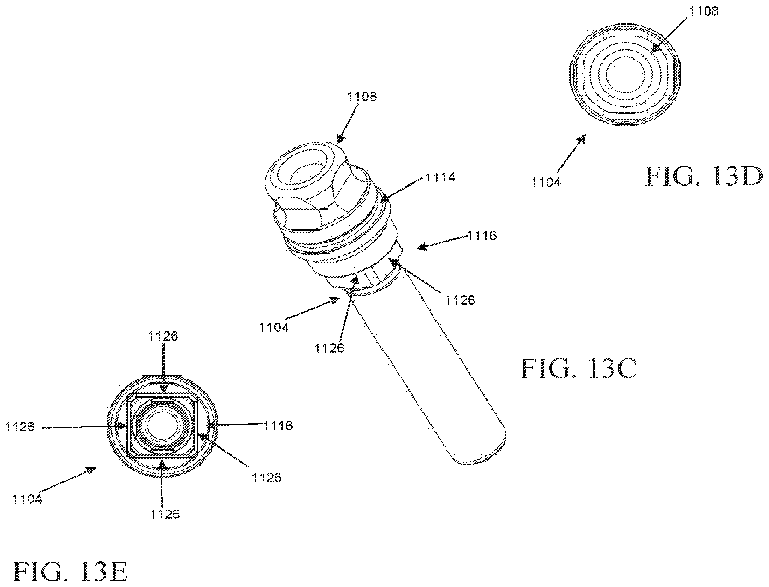

As illustrated in FIGS. 11A to 11B and 13A to 13E, the pyramid attachment structure 1108 is provided on the shaft 1104 for attachment of the assembly 1100 to a pylon or residual limb socket. The pyramid 1108 typically comprises an industry standard four-sided configuration, but in other examples, may comprise an alternative or proprietary design. The pyramid configuration may be changed by using a different shaft with a different pyramid configuration. Referring to FIGS. 13A to 13E, the pyramid 1108 is located at a first end 1110 of the shaft 1104 and may include a threaded lumen 1112 to facilitate attachment of the pyramid 1108. Next to the pyramid 1108 is an attachment region or interface 1114 of the shaft 1104 that forms a complementary interfit with the central lumen 504 of the upper housing 104. This may be a collar interface as depicted, or a thread interface, as discussed above, a bayonet mount or other type of mechanical interfit or friction fit as noted above. As depicted in FIGS. 11A to 11B, the shaft 1104 may be configured such that when assembled with the upper housing 104, the pyramid 1108 protrudes form the upper surface 128 of the upper housing 104. Adjacent to the attachment interface 1114 of the shaft 1104 may be a bore interface 1116, which may be used to grip the shaft 1104 with a wrench or pliers or other tool when coupling or decoupling the shaft 1104 and the upper housing 104.

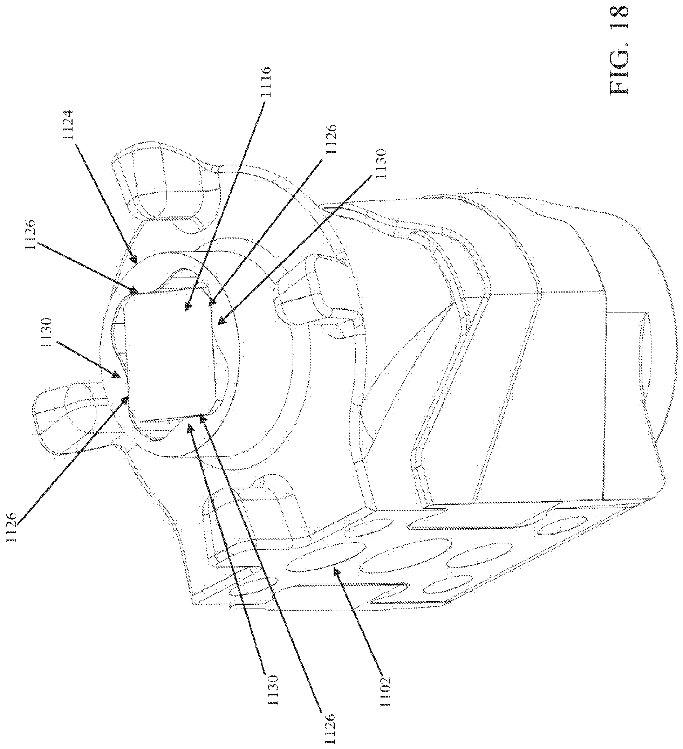

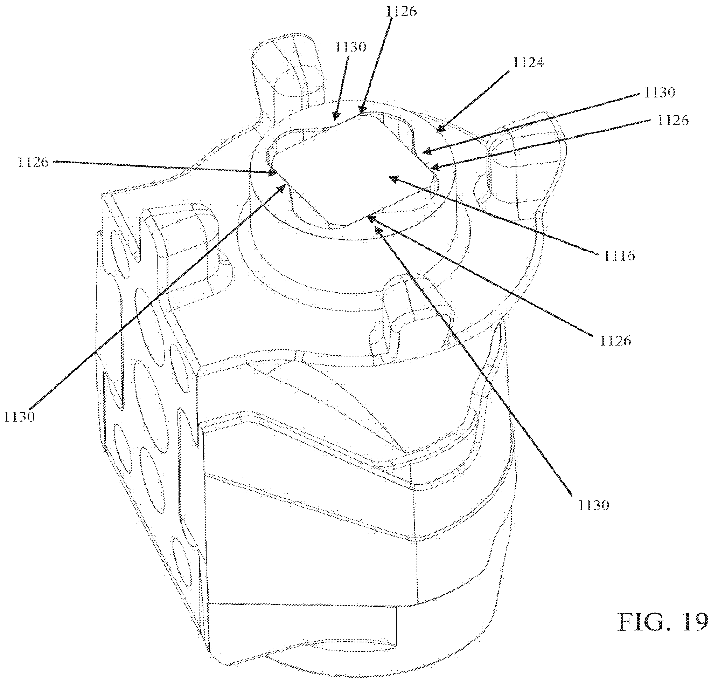

The bore interface 1116 may comprise at least one contact surface 1126 configured to contact the rounded lobes on the flange of the lower housing to restrict torsional rotation between the upper housing 104 and the lower housing 1102, as will be further discussed below. Although the contact surfaces 1126 depicted in FIGS. 13A to 13E are a rectangular interface, in other variations, the contact surfaces 1126 of the bore interface 1116 may be square or hexagonal or other polygonal shape, or may comprise a lumen in which a torque bar may be inserted to facilitate rotational coupling and decoupling of the shaft 1104 and upper housing 104. The contact surfaces 1126 of bore interface 1116 on the shaft 1104 be configured to provide a hard limit angle limit to the rotation range with respect to the lower housing 1102 as shown in FIG. 18 and 19. The contact surfaces 1126 may be configured in any suitable shaft to cooperate with the internal configuration of the flange of the lower housing 1102.

Referring back to FIGS. 13A to 13E, adjacent or inferior to the bore interface 1116 of the shaft 1104 is the body 1118 of the shaft 1104, which is configured to reside and move in the lumen 1106 of the lower housing 1102 when assembled. The length of the body 1118 of the shaft 1104 may be in the range of 1.0 inches to 7.0 inches, 2.0 inches to 5.0 inches or 2.0 inches to 4.0 inches. The diameter or cross-sectional dimension of the shaft 1104 may be in the range of 0.12 inches to 1.5 inches, 0.25 inches to 1.25 inches or 0.3 inches to 0.9 inches. In one embodiment, the diameter of the shaft may be approximately 0.55 inches and the length of the body of the shaft may be approximately 3.83 inches. Different lengths of the shaft 1104 may also be provided in order to accommodate different patient preferences, height, and functional levels, with corresponding different heights of the resilient body 102.

The second or lower end 1120 of the shaft 1104 is sized and configured to extend out from the lumen 1106 of the lower housing 1102. A retention member or assembly 110 may be attached to the second end 1120 to resist pullout of the shaft 1104 from the lower housing 1102, but may be configured to permit some vertical displacement of the shaft 1104 within the lumen 1106. This acts as a shock absorber as the upper housing 104 and lower housing 1102 resiliently compress the resilient body 102. In this particular example, the retention assembly 110 is attachable to the second end 1120 of the shaft 1104 by a closed threaded lumen 1122, but in other variations, may be attached via a clevis pin or other coupling interface. The retention assembly 110 is also configured to permit the shaft 1104 to rotate within the lumen 1106 and thereby to permit axial rotation.

In the particular examples depicted in FIGS. 1A to 1H, the axial rotation is limited by the increasing resistance to rotation provided by rotational compression of the resilient body 102 between the inferior and superior projections 116, 126. In other variations, however, the retention member or assembly 110, the contact surfaces of bore interface on the shaft 108 and the rounded lobes on the flange of the lower housing 106 may be configured to provide a hard limit angle limit to the rotation range.

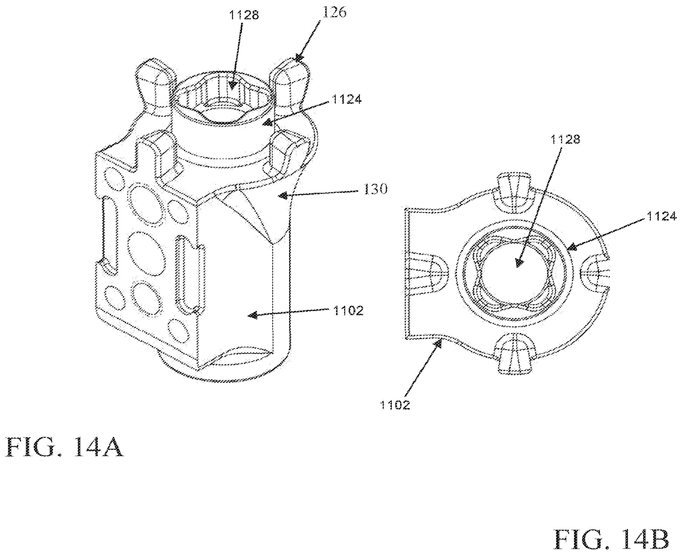

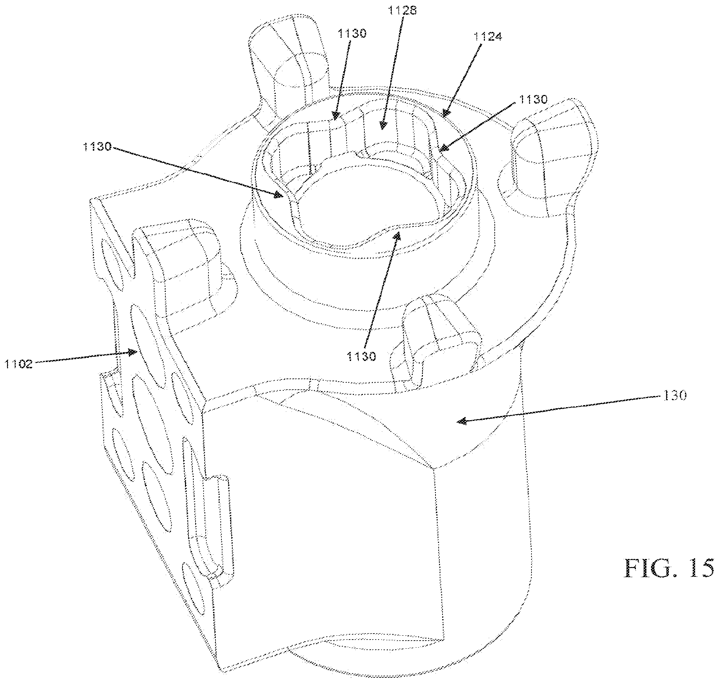

In the embodiment depicted in FIGS. 14A, 14B, and 15, the lower housing 1102 may comprise an annular projection or flange 1124. The remainder of the components for the lower housing 1102 are similar to those described above regarding lower housing 106.

The annular flange 1124 is spaced radially inward from the peripheral surface 130 and the projections 126, surrounding the longitudinal lumen 1106 of the lower housing 1102. This flange 1124 may be configured to insert or reside inside the central lumen 204 of the resilient body 102. In some variations, the annular flange 1124 may reduce the risk of eccentric displacement of the resilient body 102 during various compression and rotational movements, and may also limit the radially inward bulging of the inner surface 206 during vertical compression, and/or may act as barrier reduce the intrusion of debris and liquid into the lumen 1106 of the lower housing 1102.

The flange 1124 also provides additional support for longer tubular bearings that might be used in the lumen 1106. The use of a longer bearing may augment or reduce resistance that may be generated by off-axis forces or forces transverse to the longitudinal shaft 1104 and lumen 1106. This may also improve bearing life and tactile prosthesis response. In embodiments comprising a tubular bearing, the ratio of the bearing length to the bearing inner diameter may in the range of 1.5:1 and 10:1, or 2:1 to 6:1 or 3:1 to 5:1. The flange 1124 also allows the resilient member to be placed lower in the overall prosthesis, relative to the lumen 1106, which can shorten the build height of the prosthesis, which allows the use of the prosthesis across a greater range of residual limb lengths. Depending on the height of the annular flange 1124, the flange 1124 may also provide a hard compression stop if the amount of vertical compression results in the annular flange 1124 abutting against the inferior surface 120 of the upper housing 104. In some variations, the height of the annular flange 1124 is the range of 0.02 inches to 1.5 inches, 0.1 inches to 0.5 inches or 0.12 inches to 0.3 inches. The wall thickness of the flange 1124 may be in the range of 0.04 inches to 0.5 inches, 0.07 inches to 0.3 inches or 0.1 inches to 0.2 inches. The inner diameter may be 0.25 inches to 1.5 inches, 0.3 inches to 1.0 inches or 0.5 inches to 0.75 inches, and the outer diameter may be 0.3 inches to 2.0 inches, 0.4 inches to 1.5 inches or 0.5 inches to 1.0 inches. In one embodiment, the height of the flange may be approximately 0.47 inches and the outside diameter may be approximately 0.92 inches. In one embodiment, the lobed design the flange 1124 wall thickness may be irregular within the range of 0.16 to 0.60 inches and the inside of the lobed feature has an inscribed circle diameter of approximately 0.599 inches at minimum to approximately 0.800 inches at maximum.

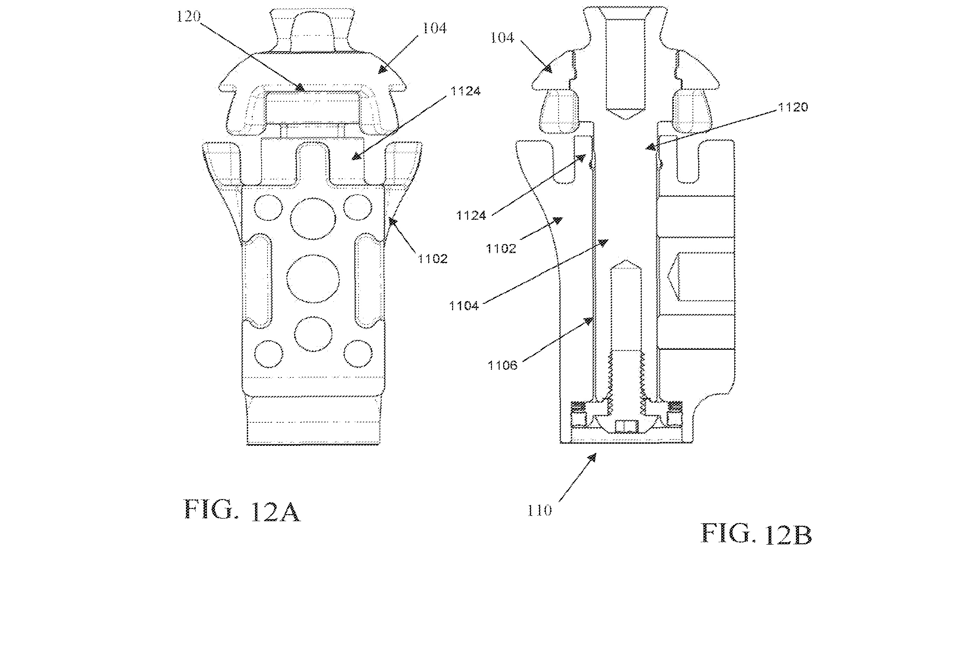

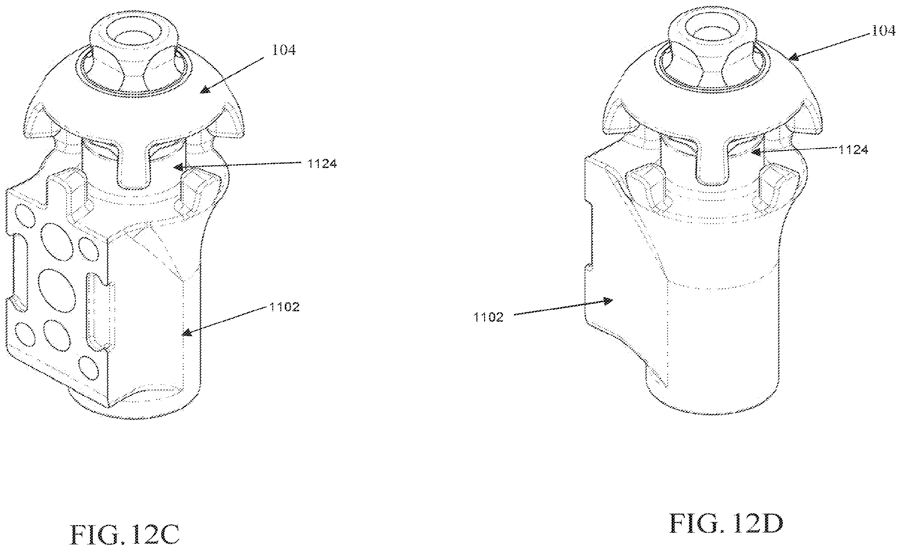

FIGS. 12A to 12D illustrate the assembled configuration of the upper housing 104, lower housing 1102 and shaft 1104, without the resilient body 108. The shaft 1104 may be configured such that the bore interface 1116 is located generally at the level of the longitudinal location of the resilient body 102. The gap or distance between the lower surface 120 of the upper housing 104 and the superior surface of the lower housing 1102 may be equal to the unstrained height of the resilient body 102. In other examples, the gap or distance may be smaller than the unstrained height of the resilient body 102, such that when assembled, the upper and lower housings 104, 1102 place the resilient body 102 in vertical compression at baseline. This baseline compressed configuration may make the haptic feel of the assembly to be more linear or predictable compared to a baseline configuration that is not compressed or where the housing gap is greater than the unstrained height of the resilient body 102.

Referring now to FIGS. 14 and 15 the flange 1124 may comprise an internal bore 1128 having a plurality of rounded lobes 1130. The rounded lobes 1130 and the contact surfaces 1126 of the bore interface 1116 on the shaft 1104 are configured to limit the rotation of the upper housing 104 with respect to the lower housing 1102. The rounded lobes 1130 are spaced apart and located opposite of one another on the internal bore 1128. In one embodiment the internal bore 1128 may comprise four rounded lobes, that are configured to contact the four contact surfaces 1126 of bore interface 1116 on the shaft 1104.

In various embodiments, the contact surfaces 1126 of bore interface 1116 on the shaft 1104 and the rounded lobes 1130 on the flange 1124 of the lower housing 1104 may be configured to provide a hard limit angle limit to the rotation range as shown in FIG. 18 and 19. In one embodiment, the angle limit of rotation is approximately 15° in the clockwise and counterclockwise directions for a total range of rotation of approximately 30°. In various embodiments, the number of contact surfaces 1126 of bore interface 1116 on the shaft 1104 are the same as the rounded lobes 1130 on the flange 1124.

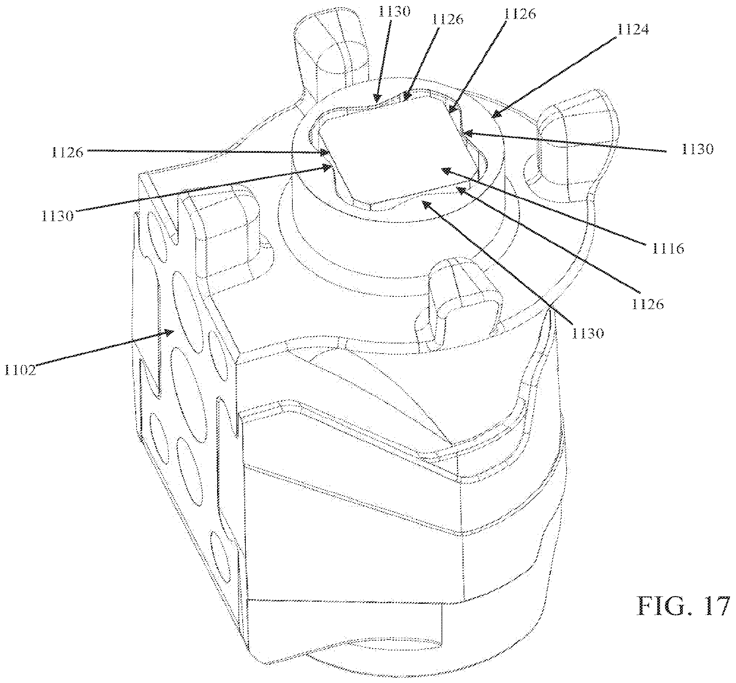

FIG 17 shows the lower housing 1102 with a portion of the shaft 1104 placed within the lumen 1106 and the shaft 1104 in the neutral position. The contact surfaces 1126 of the bore interface 1116 are not in contact with the rounded lobes 1130 on the flange 1124 of the lower housing 1102.

FIG. 18 is a side perspective view of the lower housing 1102 with a portion of the shaft 1104 placed within the lumen 1106 and the shaft 1104 rotated clockwise from the neutral position. The contact surfaces 1126 of the bore interface 1116 are in contact with the rounded lobes 1130 on the flange 1124 of the lower housing 1102 to resist torsional rotation of the upper housing 104 attached to the shaft 1104 with regard to the lower housing.

FIG. 19 is a side perspective view of the lower housing 1102 with a portion of the shaft 1104 placed within the lumen 1106 rotated counterclockwise from the neutral position. The contact surfaces 1126 of the bore interface 1116 are in contact with the rounded lobes 1130 on the flange 1124 of the lower housing 1102 to resist torsional rotation of the upper housing 104 attached to the shaft 1104 with regard to the lower housing.

The specific examples and descriptions herein are exemplary in nature and variations may be developed by those skilled in the art based on the material taught herein without departing from the scope of the present subject matter.

The technology has been described with reference to specific exemplary embodiments. Various modifications and changes, however, may be made without departing from the scope of the present technology. The description and figures are to be regarded in an illustrative manner, rather than a restrictive one and all such modifications are intended to be included within the scope of the present technology. Accordingly, the scope of the technology should be determined by the generic embodiments described and their legal equivalents rather than by merely the specific examples described above. For example, the steps recited in any method or process embodiment may be executed in any order, unless otherwise expressly specified, and are not limited to the explicit order presented in the specific examples. Additionally, the components and/or elements recited in any apparatus embodiment may be assembled or otherwise operationally configured in a variety of permutations to produce substantially the same result as the present technology and are accordingly not limited to the specific configuration recited in the specific examples.

Benefits, other advantages and solutions to problems have been described above with regard to particular embodiments; however, any benefit, advantage, solution to problems or any element that may cause any particular benefit, advantage or solution to occur or to become more pronounced are not to be construed as critical, required or essential features or components.

As used herein, the terms "comprises," "comprising," or any variation thereof, are intended to reference a non-exclusive inclusion, such that a process, method, article, composition or apparatus that comprises a list of elements does not include only those elements recited, but may also include other elements not expressly listed or inherent to such process, method, article, composition or apparatus. Other combinations and/or modifications of the above-described structures, arrangements, applications, proportions, elements, materials or components used in the practice of the present technology, in addition to those not specifically recited, may be varied or otherwise particularly adapted to specific environments, manufacturing specifications, design parameters or other operating requirements without departing from the general principles of the same.

Furthermore, in understanding the scope of the present invention, the term "comprising" and its derivatives, as used herein, are intended to be open ended terms that specify the presence of the stated features, elements, components, groups, and/or steps, but do not exclude the presence of other unstated features, elements, components, groups, and/or steps. The foregoing also applies to words having similar meanings such as the terms, "including," "having" and their derivatives. Any terms of degree such as "substantially," "about" and "approximate" as used herein mean a reasonable amount of deviation of the modified term such that the end result is not significantly changed. For example, these terms can be construed as including a deviation of at least ±5% of the modified term if this deviation would not negate the meaning of the word it modifies.

The present technology has been described above with reference to a preferred embodiment. However, changes and modifications may be made to the preferred embodiment without departing from the scope of the claims. These and other changes or modifications are intended to be included within the scope of the following claims.

## Claims

1. A prosthetic assembly, comprising:
a resilient undulating body (102) comprising an outer perimeter, a first surface (200), a second surface (202) opposite the first surface (200), and an interior opening (204) therebetween;
a longitudinal shaft (1104) located in the interior opening (204) of the undulating member (102), the shaft (1104) comprising a bore interface (1116) with at least one contact surface (1126);
a first prosthesis body (104) coupled to the longitudinal shaft (1104) and contacting the first surface (200) of the undulating member (102); and
a second prosthesis body (1102) contacting the second surface (202) of the undulating member (102) and comprising:
a longitudinal lumen (1106); and
a flange (1124) located on an upper surface of the second prosthesis body comprising an internal bore (1128) with at least one lobe (1130),
wherein the longitudinal shaft (1104) is movably located in the longitudinal lumen (1106) and wherein the at least one contact surface (1126) contacts the at least one lobe (1130) to limit the rotation of the first prosthesis body (104) with respect to the second prosthesis body (1102).

2. The prosthetic assembly of claim 1, wherein the at least one contact surface (1126) of the bore interface (1116) comprises multiple contact surfaces (1126); wherein preferably the multiple contact surfaces (1126) comprise a rectangular shape.

3. The prosthetic assembly of claim 2, wherein the at least one lobe (1130) on the internal bore (1128) comprises multiple lobes (1130); and the multiple lobes (1130) are preferably configured to contact the contact surfaces (1126) of the bore interface (1116) to resist torsional rotation of the first prosthesis body (104) with respect to the second prosthesis body (1102).

4. The prosthetic assembly of claim 1, wherein the undulating body (102) comprises a first plurality of recesses (218) on the first surface (200) of the undulating body (102).

5. The prosthetic assembly of claim 4, wherein the first prosthesis body (104) comprises a first plurality of projections (116) configured to form a mechanical interfit with the first plurality of recesses (218) of the undulating body (102).

6. The prosthetic assembly of claim 5, wherein the undulating body (102) comprises a second plurality of recesses (220) on the second surface (202) of the undulating body (102); and wherein preferably the first plurality of recesses (218) are rotationally offset from the second plurality of recesses (220) when no net rotational forces are acting on the undulating body (102); and each recess of the first plurality of recesses (218) and the second plurality of recesses (220) comprises an outer perimeter opening region, a radially inward wall opposite the outer perimeter opening, and opposing first and second side walls flanking the radially inward wall.

7. The prosthetic assembly of claim 6, wherein the second prosthesis body (1102) comprises a second plurality of projections (126) configured to form a mechanical interfit with the second plurality of recesses (200) of the undulating body (102).

8. The prosthetic assembly of claim 6, wherein:
the first plurality of recesses (218) comprises an equal angular spacing relative to a central axis of the undulating body (102); and
the second plurality of recesses (220) comprises an equal angular spacing relative to the central axis of the undulating body (102).

9. The prosthetic assembly of claim 6, wherein the angular spacing of the first plurality of recesses (218) and the angular spacing of the second plurality of recesses (220) are 90 degrees; and wherein preferably the first and second pluralities of recesses (218, 220) are offset by 40 to 65 degrees.

10. The prosthetic assembly of claim 1, wherein the undulating body (102) further comprises an internal seal extending from the second surface (202) of the undulating body (102) that is radially offset from the outer perimeter (208) of the undulating member (102) and surrounding the interior opening (204) of the undulating body (102).

11. The prosthetic assembly of claim 6, wherein the first and/or second plurality of recesses (218, 220) each comprises four recesses.

12. The prosthetic assembly of claim 1, wherein the second prosthesis body (1102) is configured to permit axial and rotational movement of the longitudinal shaft (1104) in the longitudinal lumen (1106) of the second prosthesis body (1102); and the prosthetic assembly preferably further comprises a shaft retainer (110) removably attached to the shaft (1104), and configured to resist separation of the longitudinal shaft (1104) and the second prosthesis body (1102).

13. The prosthetic assembly of claim 12, wherein the shaft retainer (110) comprises:
a removable fastener (800) configured to removably attach to the longitudinal shaft (1104);
an annular seal (806) configured to slidably seal the shaft retainer to the second prosthesis body (1102); and
a retaining washer (802) with a circumferential recess in which the annular seal (806) partially resides.

14. The prosthetic assembly of claims 12 or 13, wherein the shaft retainer (110) further comprises a spring (812); and wherein the spring (812) is preferably configured to maintain partial compression of the resilient body (102) when the prosthetic assembly is in an unloaded state.

15. The prosthetic assembly of claim 1, wherein the longitudinal shaft (1104) comprises a transverse stop surface located between a first end and a second end of the longitudinal shaft (1104), and configured to displaceably abut against a corresponding stop surface of the second prosthesis body.

## Patentansprüche

1. Prothesenanordnung, die Folgendes aufweist:
einen elastischen welligen Körper (102) der einen äußeren Umfang, eine erste Oberfläche (200), eine der ersten Oberfläche (200) entgegengesetzte zweite Oberfläche (202) und eine innere Öffnung (204) dazwischen aufweist;
einen längs verlaufenden Schaft (1104), der sich in der inneren Öffnung (204) des welligen Elements (102) befindet, wobei der Schaft (1104) eine Bohrungsschnittstelle (1116) mit mindestens einer Kontaktfläche (1126) aufweist;
einen ersten Prothesenkörper (104), der mit dem längs verlaufenden Schaft (1104) gekoppelt ist und mit der ersten Oberfläche (200) des welligen Elements (102) in Kontakt ist; und
einen zweiten Prothesenkörper (1102), der mit der zweiten Oberfläche (202) des welligen Elements (102) in Kontakt ist und Folgendes aufweist:
ein längs verlaufendes Lumen (1106); und
einen Flansch (1124), an einer oberen Oberfläche des zweiten Prothesenkörpers befindlich, der eine Innenbohrung (1128) mit mindestens einem Nocken (1130) aufweist,
wobei der längs verlaufende Schaft (1104) beweglich in dem längs verlaufenden Lumen (1106) liegt und wobei die mindestens eine Kontaktfläche (1126) mit dem mindestens einen Nocken (1130) in Kontakt ist, um die Drehung des ersten Prothesenkörpers (104) in Bezug auf den zweiten Prothesenkörper (1102) zu begrenzen.

2. Prothesenanordnung nach Anspruch 1, wobei die mindestens eine Kontaktfläche (1126) der Bohrungsschnittstelle (1116) mehrere Kontaktflächen (1126) aufweist; wobei vorzugsweise die mehreren Kontaktflächen (1126) eine rechteckige Form aufweisen.

3. Prothesenanordnung nach Anspruch 2, wobei der mindestens eine Nocken (1130) an der Innenbohrung (1128) mehrere Nocken (1130) aufweist; und die mehreren Nocken (1130) vorzugsweise so konfiguriert sind, dass sie mit den Kontaktflächen (1126) der Bohrungsschnittstelle (1116) in Kontakt sind, um einer Torsionsverdrehung des ersten Prothesenkörpers (104) in Bezug auf den zweiten Prothesenkörper (1102) zu widerstehen.

4. Prothesenanordnung nach Anspruch 1, wobei der wellige Körper (102) eine erste Mehrzahl von Aussparungen (218) an der ersten Oberfläche (200) des welligen Körpers (102) aufweist.

5. Prothesenanordnung nach Anspruch 4, wobei der erste Prothesenkörper (104) eine erste Mehrzahl von Vorsprüngen (116) aufweist, die zum Bilden einer mechanischen Passung mit der ersten Mehrzahl von Aussparungen (218) des welligen Körpers (102) konfiguriert sind.

6. Prothesenanordnung nach Anspruch 5, wobei der wellige Körper (102) eine zweite Mehrzahl von Aussparungen (220) an der zweiten Oberfläche (202) des welligen Körpers (102) aufweist; und wobei vorzugsweise die erste Mehrzahl von Aussparungen (218) gegenüber der zweiten Mehrzahl von Aussparungen (220) in Drehrichtung versetzt sind, wenn keine resultierenden Drehkräfte auf den welligen Körper (102) wirken; und jede Aussparung der ersten Mehrzahl von Aussparungen (218) und der zweiten Mehrzahl von Aussparungen (220) eine Öffnungsregion am äußeren Umfang, eine radial einwärts liegende Wand, die der Öffnung am äußeren Umfang entgegengesetzt ist, und einander gegenüberliegende erste und zweite Seitenwände, die die radial einwärts liegende Wand flankieren, aufweist.

7. Prothesenanordnung nach Anspruch 6, wobei der zweite Prothesenkörper (1102) eine zweite Mehrzahl von Vorsprüngen (126) aufweist, die zum Bilden einer mechanischen Passung mit der zweiten Mehrzahl von Aussparungen (200) des welligen Körpers (102) konfiguriert ist.

8. Prothesenanordnung nach Anspruch 6, wobei:
die erste Mehrzahl von Aussparungen (218) eine gleichmäßige Winkelweite relativ zu einer Mittelachse des welligen Körpers (102) aufweist; und
die zweite Mehrzahl von Aussparungen (220) eine gleiche Winkelweite relativ zur Mittelachse des welligen Körpers (102) aufweist.

9. Prothesenanordnung nach Anspruch 6, wobei die Winkelweite der ersten Mehrzahl von Aussparungen (218) und die Winkelweite der zweiten Mehrzahl von Aussparungen (220) 90 Grad betragen; und wobei vorzugsweise die erste und die zweite Mehrzahl von Aussparungen (218, 220) um 40 bis 65 Grad versetzt sind.

10. Prothesenanordnung nach Anspruch 1, wobei der wellige Körper (102) ferner eine sich von der zweiten Oberfläche (202) des welligen Körpers (102) erstreckende innere Dichtung aufweist, die zum äußeren Umfang (208) des welligen Elements (102) radial versetzt ist und die innere Öffnung (204) des welligen Körpers (102) umgibt.

11. Prothesenanordnung nach Anspruch 6, wobei die erste und/oder die zweite Mehrzahl von Aussparungen (218, 220) jeweils vier Aussparungen aufweist.

12. Prothesenanordnung nach Anspruch 1, wobei der zweite Prothesenkörper (1102) zum Ermöglichen der Axial- und Drehbewegung des längs verlaufenden Schafts (1104) in dem längs verlaufenden Lumen (1106) des zweiten Prothesenkörpers (1102) konfiguriert ist; und die Prothesenanordnung vorzugsweise einen Schafthalter (110) aufweist, der entfernbar an dem Schaft (1104) angebracht ist und so konfiguriert ist, dass er einer Trennung des längs verlaufenden Schafts (1104) und des zweiten Prothesenkörpers (1102) widersteht.

13. Prothesenanordnung nach Anspruch 12, wobei der Schafthalter (110) Folgendes aufweist:
ein entfernbares Befestigungselement (800), das zum entfernbaren Anbringen des längs verlaufenden Schafts (1104) konfiguriert ist;
eine ringförmige Dichtung (806), die zum gleitfähigen Abdichten des Schafthalters zum zweiten Prothesenkörper (1102) konfiguriert ist; und
eine Sicherungsscheibe (802) mit einer Aussparung am Umfang, in der die ringförmige Dichtung (806) teilweise liegt.

14. Prothesenanordnung nach Anspruch 12 oder 13, wobei der Schafthalter (110) ferner eine Feder (812) aufweist; und wobei die Feder (812) vorzugsweise so konfiguriert ist, dass sie ein teilweises Zusammendrücken des elastischen Körpers (102) aufrechterhält, wenn die Prothesenanordnung in einem unbelasteten Zustand ist.

15. Prothesenanordnung nach Anspruch 1, wobei der längs verlaufende Schaft (1104) eine quer verlaufende Anschlagfläche zwischen einem ersten Ende und einem zweiten Ende des längs verlaufenden Schafts (1104) aufweist und zum verlagerbaren Anstoßen an einer entsprechenden Anschlagfläche des zweiten Prothesenkörpers konfiguriert ist.

## Revendications

1. Assemblage prothétique, comprenant :
un corps ondulant résilient (102) comprenant un périmètre externe, une première surface (200), une deuxième surface (202) opposée à la première surface (200), et une ouverture intérieure (204) entre elles,
un arbre longitudinal (1104) situé dans l'ouverture intérieure (204) du membre ondulant (102), l'arbre (1104) comprenant une interface d'alésage (1116) avec au moins une surface de contact (1126) ;
un premier corps de prothèse (104) couplé à l'arbre longitudinal (1104) et touchant la première surface (200) du membre ondulant (102) ; et
un deuxième corps de prothèse (1102) touchant la deuxième surface (202) du membre ondulant (102) et comprenant :
une lumière longitudinale (1106) ; et
une bride (1124) située sur une surface supérieure du deuxième corps de prothèse comprenant un alésage interne (1128) avec au moins un lobe (1130),
dans lequel l'arbre longitudinal (1104) est situé de manière amovible dans la lumière longitudinale (1106) et dans lequel la au moins une surface de contact (1126) touche le au moins un lobe (1130) afin de limiter la rotation du premier corps de prothèse (104) par rapport au deuxième corps de prothèse (1102).

2. Assemblage prothétique selon la revendication 1, dans lequel la au moins une surface de contact (1126) de l'interface d'alésage (1116) comprend de multiples surfaces de contact (1126), dans lequel les multiples surfaces de contact comprennent de préférence une forme rectangulaire.

3. Assemblage prothétique selon la revendication 2, dans lequel le au moins un lobe (1130) sur l'alésage interne (1128) comprend de multiples lobes (1130) ; et les multiples lobes (1130) sont configurés de préférence pour toucher les surfaces de contact (1126) de l'interface d'alésage (1116) afin de résister à une rotation en torsion du premier corps de prothèse (104) par rapport au deuxième corps de prothèse (1102).

4. Assemblage prothétique selon la revendication 1, dans lequel le corps ondulant (102) comprend une première pluralité d'évidements (218) sur la première surface (200) du corps ondulant (102).

5. Assemblage prothétique selon la revendication 4, dans lequel le premier corps de prothèse (104) comprend une première pluralité de saillies (116) configurées pour former un ajustement mécanique avec la première pluralité d'évidements (218) du corps ondulant (102).

6. Assemblage prothétique selon la revendication 5, dans lequel le corps ondulant (102) comprend une deuxième pluralité d'évidements (220) sur la deuxième surface (202) du corps ondulant (102) ; et dans lequel la première pluralité d'évidements (218) sont de préférence décalés en rotation de la deuxième pluralité d'évidements (220) lorsqu'aucune force nette de rotation n'agit sur le corps ondulant (102) ; et chaque évidement de la première pluralité d'évidements (218) et de la deuxième pluralité d'évidements (220) comprend une région d'ouverture sur le périmètre externe, une paroi radialement vers l'intérieur à l'opposé de l'ouverture sur le périmètre externe, et à l'opposé de la première et deuxième paroi latérale flanquant la paroi radialement vers l'intérieur.

7. Assemblage prothétique selon la revendication 6, dans lequel le deuxième corps de prothèse (1102) comprend une deuxième pluralité de saillies (126) configurées pour former un ajustement mécanique avec la deuxième pluralité d'évidements (200) du corps ondulant (102).

8. Assemblage prothétique selon la revendication 6, dans lequel :
la première pluralité d'évidements (218) comprend un espacement angulaire égal par rapport à un axe central du corps ondulant (102) ; et
la deuxième pluralité d'évidements (220) comprend un espacement angulaire égal par rapport à l'axe central du corps ondulant (102).

9. Assemblage prothétique selon la revendication 6, dans lequel l'espacement angulaire de la première pluralité d'évidements (218) et l'espacement angulaire de la deuxième pluralité d'évidements (220) sont de 90 degrés ; et dans lequel la première et la deuxième pluralité d'évidements (218, 220) sont de préférence décalées de 40 à 65 degrés.

10. Assemblage prothétique selon la revendication 1, dans lequel le corps ondulant (102) comprend en outre un joint d'étanchéité interne s'étendant de la deuxième surface (202) du corps ondulant (102) qui est décalée radialement du périmètre externe (208) du corps ondulant (102) et entourant l'ouverture intérieure (204) du corps ondulant (102).

11. Assemblage prothétique selon la revendication 6, dans lequel la première et/ou la deuxième pluralité d'évidements (218, 220) comprennent chacune quatre évidements.

12. Assemblage prothétique selon la revendication 1, dans lequel le deuxième corps de prothèse (1102) est configuré pour permettre le mouvement axial et rotatif de l'arbre longitudinal (1104) dans la lumière longitudinale (1106) du deuxième corps de prothèse (1102) ; et l'assemblage prothétique comprend de préférence en outre un dispositif de retenue d'arbre (110) attachable de manière amovible à l'arbre (1104) et configuré pour résister à la séparation de l'arbre longitudinal (1104) et du deuxième corps de prothèse (1102).

13. Assemblage prothétique selon la revendication 12, dans lequel le dispositif de retenue d'arbre (110) comprend :
un dispositif de fixation amovible (800) configuré pour attacher l'arbre longitudinal (1104) de manière amovible ;
un joint d'étanchéité annulaire (806) configuré pour sceller de manière coulissante le dispositif de retenue d'arbre au deuxième corps de prothèse (1102) ; et
une rondelle de retenue (802) ayant un évidement sur sa circonférence dans lequel le joint d'étanchéité annulaire (806) demeure partiellement.

14. Assemblage prothétique selon la revendication 12 ou 13, dans lequel le dispositif de retenue d'arbre (110) comprend en outre un ressort (812) ; et dans lequel le ressort (812) est configuré de préférence pour maintenir une compression partielle du corps résilient (102) lorsque l'assemblage prothétique est à l'état non chargé.

15. Assemblage prothétique selon la revendication 1, dans lequel l'arbre longitudinal (1104) comprend une surface de butée transversale située entre une première extrémité et une deuxième extrémité de l'arbre longitudinal (1104), et configurée pour buter en déplacement contre une surface de butée correspondante du deuxième corps de prothèse .
